# EUROPEAN PATENT SPECIFICATION

(11) **EP 3 122 416 B1**
(45) Date of publication and mention of the grant of the patent: **07.06.2023**
(21) Application number: 15768911.8
(22) Date of filing: 23.03.2015
(51) Int. Cl.: A61B 18/14, A61B 5/06, A61B 5/0215, A61M 25/00, A61B 18/00, A61M 39/08, A61M 29/02, A61B 90/00

(54) **MEDICAL APPARATUS FOR FLUID COMMUNICATION**
MEDIZINISCHE VORRICHTUNG ZUR FLUIDÜBERTRAGUNG
APPAREIL MÉDICAL POUR LA COMMUNICATION DE FLUIDE

(30) Priority: 24.03.2014 US 201414222909
(43) Date of publication of application: 01.02.2017
(60) Divisional application: 23170810.8
(73) Proprietor: Boston Scientific Medical Device Limited, Galway H91 Y868 (IE)
(72) Inventor: URBANSKI, John Paul, Toronto, Ontario M4L 2N7 (CA); SHAH, Krishan, Mississauga, Ontario L5M 2C7 (CA); MIRZA, Mahmood, Toronto, Ontario M4A 2X7 (CA); VISRAM, Naheed, Epsom Surrey KT172PJ (GB); HARTLEY, Amanda, Caledon, Ontario L7C 1B1 (CA); DAVIES, Gareth, Toronto, ON M6G 1P6 (CA)
(74) Representative: Pfenning, Meinig & Partner mbB
(86) International application number: PCT/IB2015/052118
(87) International publication number: WO 2015/145332

(56) References cited:
- EP-A2- 1 474 203
- EP-B1- 1 474 203
- WO-A1-2013/179103
- WO-A1-2013/179103
- US-A- 5 300 069
- US-A1- 2005 059 966
- US-A1- 2006 241 586
- US-A1- 2007 060 879
- US-A1- 2009 163 850
- US-A1- 2012 232 546
- US-A1- 2013 184 735
- US-A1- 2014 206 987
- US-B2- 8 388 549

## Description

### FIELD OF THE INVENTION

The present disclosure relates generally to methods and devices usable within the body of a patient to deliver fluids, withdraw fluids, and measure fluid pressure. More specifically, the present invention is concerned with medical devices with side apertures or side-ports.

### SUMMARY OF THE DISCLOSURE

In US 2012/0232546A1 a radiofrequency perforation apparatus is disclosed for creating a channel at a target location in a body of a patient, the radiofrequency perforation apparatus comprising: a substantially tubular member defining a tubular member lumen; an end member partially positioned within the tubular member lumen at a distal end of the tubular member, the end member including an electrode; and an electrical insulator extending along the substantially tubular member.
In US 2007/060879A1, a system is disclosed that drives a catheter and an implement positioned within and axially moveable relative to the catheter through an anatomical body. The system includes an implement driver which receives and couples to the implement, and a catheter driver which receives and couples to the catheter. A controller coupled to the catheter and implement drivers directs the operation of the drivers to move the implement and catheter relative to each other.
In US 2009/163850A1, a guidable catheter for delivering radiofrequency energy to treatment site of animal is disclosed, which has a guide wire and radio frequency wires extended along respective lengths of shaft, and an angulation attached to a distal end of the guide wire.
In WO 2013179103A1, a medical device is disclosed. In particular, a radiopaque-tipped radiofrequency perforation apparatus is disclosed for creating channel at e.g. atrial septum of patient, which has a marker associated with an elongated element such that fluid flow through lumen is not obstructed by the marker.
Document US5300069 A discloses further relevant background art.

The present invention is defined by the appended claims only, in particular by the scope of appended independent claim 1. References to "embodiments" throughout the description which are not under the scope of the appended claims merely represents possible exemplary executions and are therefore not part of the present invention.

This disclosure describes embodiments of a kit and its constituent components which together form an apparatus which provides fluid communication between a medical device's lumen and the surrounding environment by a conduit cooperatively defined by the medical device and a tubular member into which the device is inserted. The medical device and tubular member are configured to fit together such that an outer surface of the distal region of the medical device cooperates with an inner surface of the tubular member to define the conduit between the side-port of the medical device and a distal end of the tubular member. The conduit is operable for uses such as injecting fluid, withdrawing fluid, and measuring pressure. Methods of assembling and using the apparatus are also described.

In one broad aspect, examples of the present disclosure describe a method of establishing a conduit for fluid communication for a medical device. The method comprises the steps of (a) inserting a medical device having at least one side-port into a tubular member, and (b) cooperatively defining a conduit for fluid communication by positioning the side-port of the medical device such that a space exists between the side-port and an inner wall of the tubular member. This space forms a part of the conduit, which extends at least between the side-port and a distal end of the tubular member.

As a feature of this broad aspect, some examples further comprise a step (c) of delivering a fluid through the side-port and distally through the distal end of the tubular member.

In another broad aspect, the present disclosure describes a medical device comprising an elongate member having a closed distal end. The elongate member comprises a proximal portion, a distal portion, a device lumen, at least one side-port in fluid communication with the device lumen, and a distal tip comprising an electrode. The distal portion extends from the at least one side-port to the distal end of the elongate member. The proximal portion defines a first outer diameter larger than the substantially constant second outer diameter defined by the distal portion.

As a feature of this broad aspect, some embodiments further include an elongate member comprising an electrically conductive material coupled to the electrode, and a layer of insulation covering the electrically conductive material.

In another broad aspect, the present disclosure describes a dilator for use with a medical device, the dilator comprising a tubular member defining a lumen in fluid communication with a distal end aperture. The tubular member comprises a proximal region having a first inner diameter, and a distal region having an increased diameter portion defining a second inner diameter. The second inner diameter is greater than the first inner diameter, and substantially constant along the increased diameter portion, which extends proximally from a distal end of the dilator.

In another broad aspect, the present disclosure describes a kit comprising a tubular member defining a tubular member lumen in fluid communication with a distal end aperture, and a medical device comprising a closed distal end, a device lumen in fluid communication with at least one side-port, and a distal portion extending from the at least one side-port to a distal end of the medical device. The medical device and tubular member are configured to cooperatively form a conduit between an outer surface of the distal portion and an inner surface of the tubular member when the medical device is inserted within the tubular member lumen. The conduit extends at least between the side-port and the distal end aperture, and enables fluid communication to an environment external to the distal end aperture.

In yet another broad aspect, the present disclosure describes an apparatus comprising a tubular member defining a tubular member lumen in fluid communication with a distal end aperture, and a medical device located within the tubular member lumen. The medical device comprises a closed distal end, a device lumen in fluid communication with at least one side-port, and a distal portion extending from the at least one side-port to a distal end of the medical device. An outer surface of the distal portion of the medical device and an inner surface of the tubular member define a conduit extending at least between the side-port and the distal end aperture. The conduit enables fluid communication between the side-port and an environment external to the distal end aperture.

### BRIEF DESCRIPTION OF THE DRAWINGS

In order that the invention may be readily understood, embodiments of the invention are illustrated by way of examples in the accompanying drawings.
Figure 1 illustrates a perspective view of a medical device in accordance with an embodiment of the present disclosure;
Figures 2A to 2D illustrate partial perspective views of distal regions of embodiments of medical devices;
Figure 2E illustrates a cross-sectional view of a distal region of an embodiment of a medical device;
Figures 3A to 3D illustrate perspective views of various electrode configurations;
Figures 4A and 4B illustrate a partially cut-away side view and an end view, respectively, of a medical device and a tubular member in accordance with an embodiment of the present disclosure;
Figures 5A and 5B illustrate a partially cut-away side view and an end view, respectively, of a medical device and a tubular member in accordance with the present invention:
Figures 5C and 5D illustrate end views of a medical device and a tubular member in accordance with alternative embodiments of the present invention;
Figures 6A and 6B illustrate a partially cut-away side view and an end view, respectively, of a tubular member in accordance with another embodiment of the present disclosure;
Figures 7A and 7B illustrate a partially cut-away side view and an end view, respectively, of a medical device and a tubular member in accordance with another embodiment of the present disclosure;
Figure 8 illustrates a perspective view of a system including a medical device in accordance with the present disclosure;
Figures 9A and 9B illustrate partially cut-away views of a method using an apparatus in accordance with an embodiment of the present disclosure;
Figure 10A illustrates a perspective view of an elongate member portion of the medical device shown in Figure 1;
Figure 10B illustrates a partial perspective view of an alternative elongate member usable in the medical device shown in Figure 1;
Figure 10C illustrates a partial perspective view of another alternative elongate member usable in the medical device shown in Figure 1;
Figure 10D illustrates a partial perspective view of yet another alternative elongate member usable in the medical device shown in Figure 1;
Figure 11A illustrates a perspective view of a medical device in accordance with an yet another alternative embodiment of the present disclosure, the medical device including a curved section;
Figures 11B illustrates a partial perspective view of a medical device in accordance with yet another alternative embodiment of the present disclosure, the medical device including an alternative curved section;
Figures 11C illustrates a partial perspective view of a medical device in accordance with yet another alternative embodiment of the present disclosure, the medical device including another alternative curved section;
Figure 12A illustrates a top elevation view of an embodiment of a hub; and
Figure 12B illustrates a side cross-sectional view taken along the line 5B-5B of Figure 12A.

### DETAILED DESCRIPTION

Puncturing devices of various types are used to create punctures or channels through tissues. These devices may utilize a variety of puncturing means, for example, mechanical, electrical or optical. Typically, such devices are inserted into a patient's body through tubular devices such as dilators or sheaths. In many applications, a user may desire to inject and/or withdraw fluid through the device prior to, during, or after puncturing.

The present inventors have discovered that attempting to inject and/or withdraw fluid while such puncturing devices are held within other tubular devices may require excessive pressure and/or force (e.g. for suction or injection) due to increased resistance to fluid flow. This increased resistance is a result of the dilator or sheath partially or totally occluding the apertures on the puncturing devices.

The present inventors have conceived of, and reduced to practice, embodiments of medical devices and tubular members, e.g. dilators, configured to allow for more efficient fluid communication between a lumen of the medical device and an environment external to the dilator. This facilitates fluid transfer, pressure measurements and the like through the medical device even while the device is inserted within the tubular member.

Some embodiments of the present disclosure include a medical device with lateral apertures or side-ports which fits within a tubular member, wherein the medical device and tubular member are configured to cooperatively define a path or conduit for fluid communication between the lumen defined by the medical device and the environment outside the device and tubular member. In typical embodiments, the medical device and tubular member cooperatively form a conduit between an outer surface of the distal portion of the medical device, and an inner surface of the tubular member when the medical device is inserted within the tubular member lumen. The conduit extends at least between the side-port of the medical device and the distal end aperture of the tubular member. Embodiments of the present invention thus minimize or reduce any obstruction, blockage, or partial blockage of side-ports of such medical devices by a dilator or any ancillary device through which the medical device is placed.

Embodiments of the present invention provide for improved efficiencies in fluid communication while avoiding the necessity of defining an open, or partially open, distal aperture in the medical device (i.e. an aperture defined by a distal face/surface of the device). This helps to mitigate a concern of cutting a plug of tissue (often referred to as 'coring' the tissue) when creating a puncture using, for example, electrical energy with a circular, open-ended electrode. If an open-ended or open-faced ring electrode is used to cut tissue, a core (or plug) of tissue can be cut from the tissue and subsequently captured in the lumen of the device. The tissue core may then be released from the lumen by flushing or other means, potentially leading to emboli and increasing the risk of a stroke or other ischemic event. Embodiments of the present invention allow for fluid communication with an external environment without requiring an open distal end on the medical device, thereby obviating the concern of creating these embolic particles.

In addition, embodiments of the present invention allow for larger electrodes to be used for cutting or puncturing tissues, as described herein below. Other advantages and benefits of embodiments of the present invention will be apparent to those of skill in the art.

With specific reference now to the drawings in detail, it is stressed that the particulars shown are by way of example and for purposes of illustrative discussion of embodiments of the present invention only. In this regard, no attempt is made to show structural details of the invention in more detail than is necessary for a fundamental understanding of the invention. The description taken with the drawings will make apparent to those skilled in the art how the several forms of the invention may be embodied in practice.

It herein is for the purpose of description and should not be regarded as limiting.

As used herein, the terms 'proximal' and 'distal' are defined with respect to the user. That is, the term 'proximal' refers to a part or portion closer to the user, and the term 'distal' refers to a part or portion further away from the user when the device is in use. Also, it should be noted that while, for clarity of explanation, the term tubular or tubular member is used to describe the members that enclose the disclosed medical devices, the term tubular member is intended to describe both circular and non-circular embodiments of the enclosing member. The term tubular member is used in this disclosure to describe dilators, sheaths, and other members that define a lumen for containing a medical device.

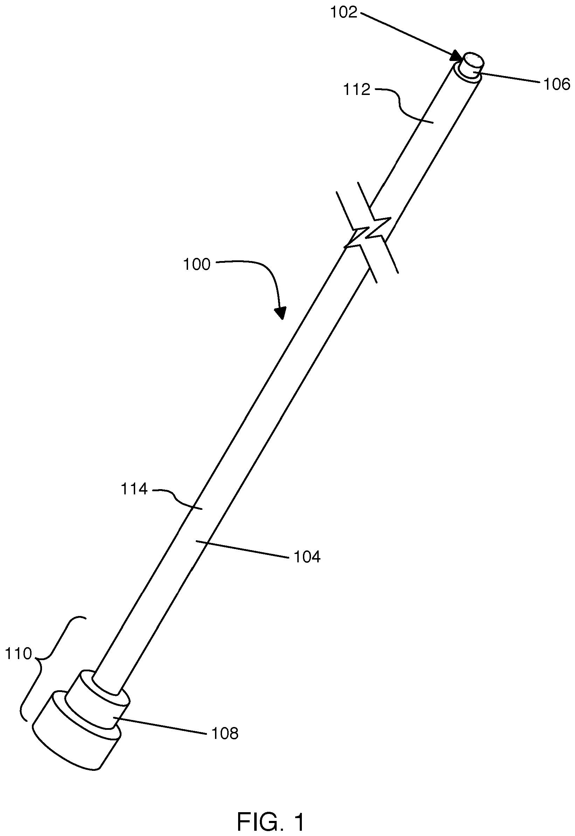

Referring to Figure 1, there is shown a medical device 100 in accordance with the present disclosure.

The medical device 100 is usable for creating a channel at a target location in a body of a patient. The medical device 100 includes a handle 110, a distal section 112 and a force transmitting section 114 extending between the distal section 112 and the handle 110. The distal section 112 defines a distal section length, and includes an electrode 106 and an electrical insulator 104 extending proximally from the electrode 106.

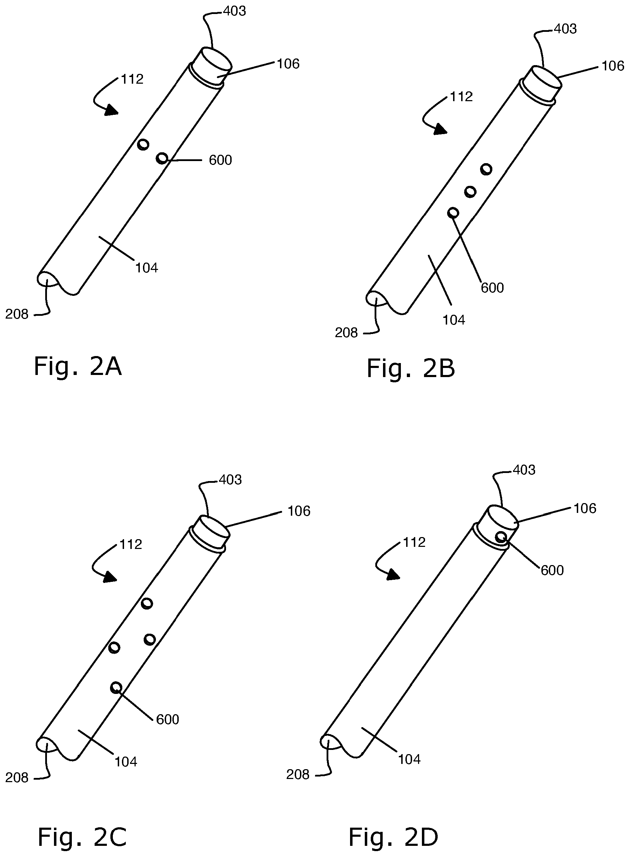

Typically, the medical device 100 includes an electrically conductive elongate member 102 having an electrical insulator 104 disposed thereon. The electrical insulator 104 substantially covers the entire outer surface of the elongate member 102 such that elongate member 102 is able to deliver energy from its proximal region to the electrode 106 at its distal region, without substantial leakage of energy along the length of the elongate member 102. The elongate member 102 defines a lumen 208 and at least one side-port 600 (shown, for example, in Figures 2A to 2D), which is in fluid communication with the lumen 208.

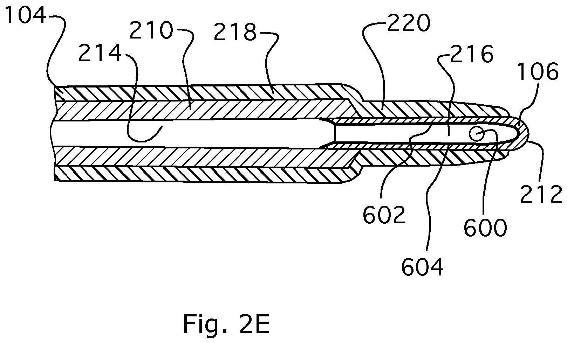

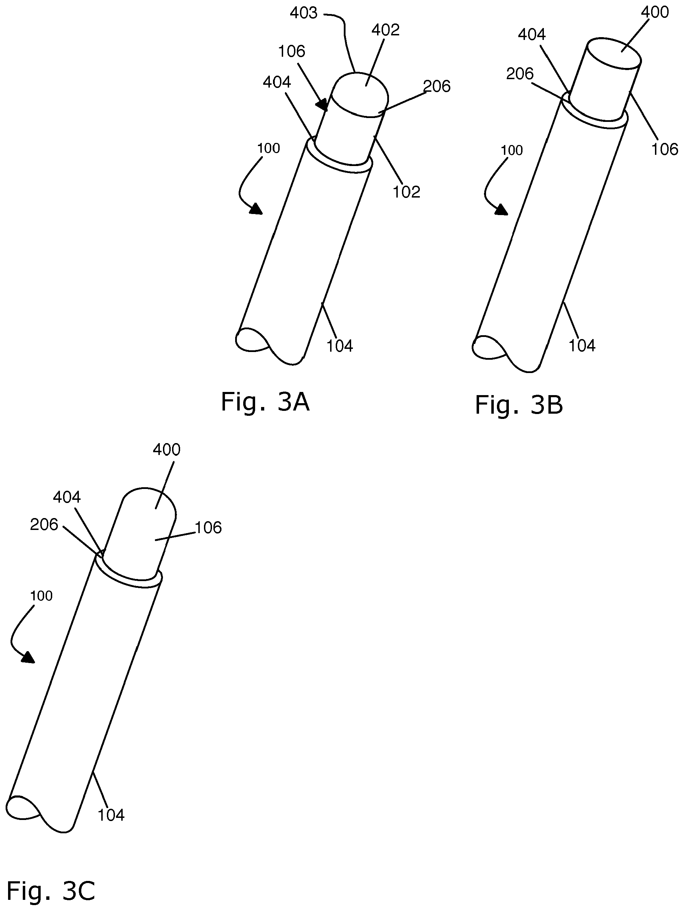

The one or more side-ports 600 are particularly useful in typical embodiments of medical device 100 wherein a lumen 208 of the elongate member 102 is not open to the surrounding environment via the distal end of the medical device 100 (i.e. wherein medical device 100 is a close-ended device), for example, in the embodiments of Figures 2E, and 3A to 3C. In such embodiments, the lumen 208 extends substantially longitudinally through the force transmitting section 114, and through a section of the distal section 112, and terminates in the distal section 112 at a location substantially spaced apart from the distal tip 403, such that the distal tip 403 remains closed.

In embodiments comprising side-port(s) 600, the side-port(s) 600 allow for fluids to be injected into the surrounding environment from the lumen 208, and/or allow for pressure to be measured by providing a pressure transmitting lumen through medical device 100. In some examples, the side-port(s) 600 are formed radially through elongate member 102 and electrical insulator 104, thereby allowing for fluid communication between the surrounding environment and the lumen 208. In alternative embodiments, a side-port 600 is formed radially through a portion of the electrode 106.

The size and shape of the side-port(s) 600 may vary depending on the intended application of the medical device 100. For example, in one embodiment, the side-port(s) 600 is between about 0.25 mm and about 0.45 mm in diameter. Some embodiments include side-ports of more than one size. In addition, the number of side-ports 600 may vary, and they may be located anywhere along the medical device 100 that does not interfere with the functioning of the device. For example, as shown in Figure 2A, the medical device 100 includes two side-ports 600 located about 1 cm from the distal end of the elongate member 102, at substantially the same longitudinal position along the elongate member 102. In another embodiment, as shown in Figure 2B, the medical device 100 includes about 3 side-ports located at the same circumferential position and spaced longitudinally at about 1.0cm, 1.5cm, and 2.0cm from the distal end of the elongate member 102. In another embodiment, as shown in Figure 2C, the side-ports 600 are staggered, such that they are spaced apart both circumferentially as well as longitudinally. In a further embodiment, as shown in Figure 2D, the side-ports 600 are located on the electrode 106. In some embodiments, the side-port(s) 600 have a smooth or rounded wall, which serves to minimize or reduce trauma to bodily tissue. For example, some such embodiments comprise one or more side-port(s) 600 with a smooth outer circumferential edge created by sanding the circumferential edges to a smooth finish or, for example, by coating the edges with a lubricious material.

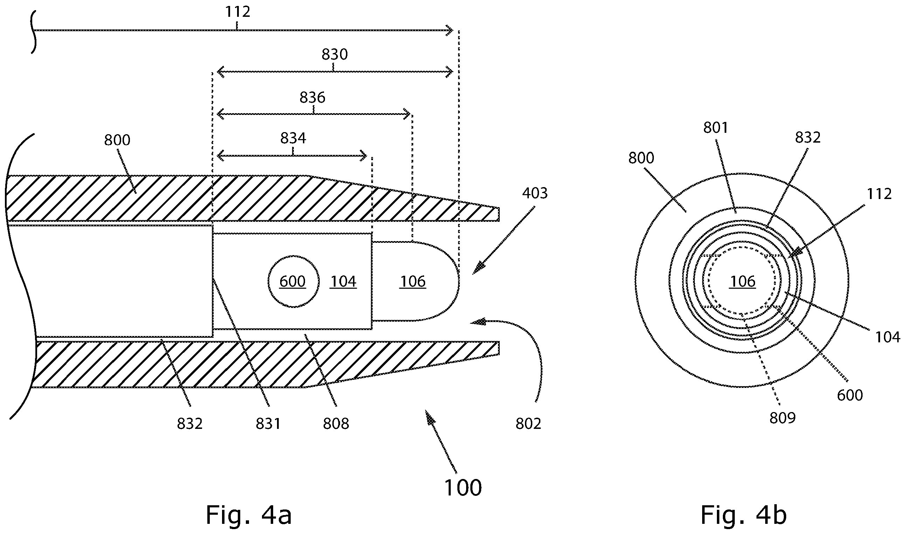

As previously described, when a medical device that relies on side-ports to provide fluid communication between its lumen and the surrounding environment is inside a lumen of a close fitting member, the side-ports may be partially or completely occluded or blocked. The embodiments of Figures 4 to 9 relate to an apparatus that provides an effective conduit from the lumen of medical device to the environment outside of the device, and methods of using such apparatus.

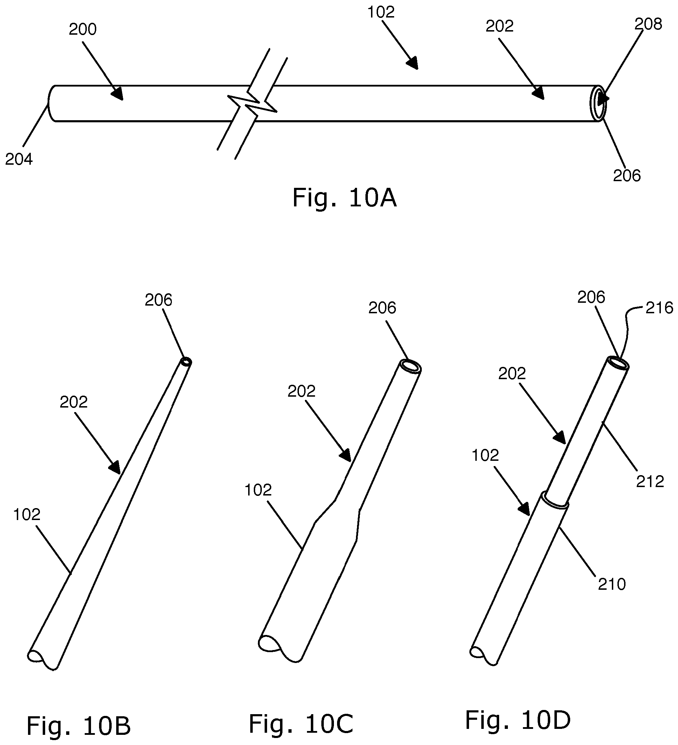

Figures 4A and 4B illustrate a partially cut-away side view and an end view, respectively, of a distal section 112 of medical device 100 positioned within tubular member 800. As described in more detail herein below, some embodiments of medical device 100 are comprised of a single piece elongate member 102 (as shown in Figure 1 and Figure 10A) and some other embodiments of medical device 100 are comprised of two elongate members, main member 210 and end member 212, which are joined together (as shown in Figures 10D and 2E). Depending on the embodiment of medical device 100 being considered, distal section 112 may be the distal section of a single piece elongate member 102, the distal section of an end member 212, or the distal section of some other embodiment of medical device 100. In Figures 4 to 9, the lumen defined by distal section 112 may be either lumen 208 of elongate member 102 or end member lumen 216. For descriptive purposes, the lumen defined by distal section 112 in Figures 4 to 9 is referred to as device lumen 809.

Tubular member 800 may comprise a dilator, a sheath, or some other member defining a lumen configured to receive a medical device 100.

Referring to Figures 4A and 4B, illustrated features of an embodiment of distal section 112 of medical device 100 include a change in diameter 831, a distal portion 830, device lumen 809 defined by a body of the medical device 100, a side-port 600 in fluid communication with the lumen, and a closed distal end. Distal portion 830 has an outer diameter less than the outer diameter of distal section 112 proximal of the change in diameter 831, i.e., distal portion 830 has a reduced diameter. In the embodiment of Figure 4A, distal tip 403 of the medical device comprises a distal electrode 106. Some alternative embodiments of medical device 100 do not include an electrode. Tubular member 800 defines tubular member lumen 802. Tubular member 800 and distal portion 830 of medical device 100, in combination, define conduit 808 whereby medical device 100 is able to provide sufficient fluid flow for delivering contrast fluid to stain tissue. Fluid (e.g. blood) may also be withdrawn through the path defined by conduit 808, side-port 600, and device lumen 809. In the example of Figure 4A, conduit 808 includes the space between tubular member 800 and reduced diameter distal portion 830, and the portion of tubular member lumen 802 distal of medical device 100.

In the embodiment of Figure 4A, distal portion 830 is distal of change in diameter 831 and includes insulated part 834 and electrode 106. Constant diameter part 836 is distal of change in diameter 831 and includes insulated part 834 and the straight longitudinal part of electrode 106 that has a constant diameter (i.e. the portion of electrode proximal of the dome shaped electrode tip). Constant diameter part 836 of distal portion 830 does not taper and may be described as having a substantially constant diameter longitudinally. There is a minor change in outer diameter at the distal end of electrical insulator 104, but with regards to fluid flow, it can be considered negligible.

In the embodiment of Figure 4A, a small space or gap 832 exists between the tubular member 800 and the part of distal section 112 proximal of the change in diameter 831. It is common for embodiments of medical device 100 and tubular member 800 to have a small gap 832 between the outer diameter of medical device and the inner diameter of tubular member. Completely eliminating the gap would result in increased friction between the medical device and tubular member and could result in difficulty advancing medical device 100 through tubular member 800. In typical embodiments, the gap is small enough that it prevents a substantial flow of fluids such as contrast fluids, which are typically 3 to 5 times more viscous than water.

In the embodiment of Figure 4A, side-port 600 is close to the change in diameter 831 whereby the larger diameter part of distal section 112 functions as a brace to keep tubular member 800 from blocking side-port 600. Figure 4A illustrates an abrupt change in diameter. Alternative embodiments have a less abrupt change in diameter. Typical embodiments of medical device 100 include a second side-port, with the two side-ports being opposite to each other. Some alternative embodiments include more than two side-ports. Other alternative embodiments have one side-port. In some alternative embodiments of medical device 100, side-port 600 is longitudinally elongated, i.e., capsule-shaped.

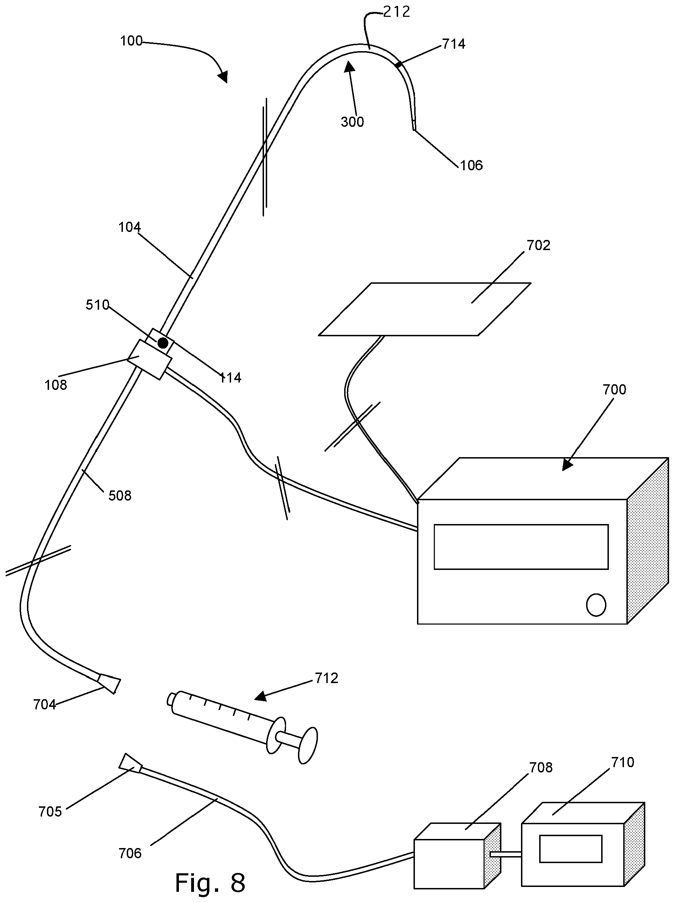

The side-port(s) 600 and the device lumen 809 together provide a pressure transmitting lumen. The pressure transmitting lumen is operable to be coupled to a pressure transducer, for example, external pressure transducer 708 (to be described with respect to Figure 8).

Distal tip 403 of medical device 100 is shown in the example of Figure 4A as being slightly proximal of the distal end of tubular member 800. In this position, fluid communication between the medical device lumen and the surrounding environment may be established. Fluid communication may also be established when distal tip 403 is positioned further proximal of the distal end of tubular member 800, when distal tip 403 is aligned with the distal end of tubular member 800, and when distal tip 403 is positioned distal of the distal end of tubular member 800. If distal tip 403 is positioned such that side-port 600 is distal of the distal end of tubular member 800, it is still possible to deliver fluid in a radial direction.

Typical embodiments of medical device 100 comprise a conductive member (elongate member 102, or main member 210 joined to end member 212), which is typically comprised of a metallic material. The conductive member is in electrical communication with distal electrode 106, and a layer of insulation (electrical insulator 104) covers the metallic material. In other words, the elongate member 102 comprises an electrically conductive material, and a layer of insulation covers the electrically conductive material, the electrically conductive material being electrically coupled to the electrode 106. For some single piece embodiments, elongate member 102 has an on outer diameter proximal of change in diameter 831 of about 0.7 mm to about 0.8 mm at distal end 206, and an outer diameter for reduced diameter distal portion 830 of about 0.4 mm to about 0.62 mm. For some two piece embodiments, end member 212 has an outer diameter proximal of change in diameter 831 of about 0.40 mm to about 0.80 mm, and an outer diameter for distal portion 830 of about 0.22 mm to about 0.62 mm. The above described embodiments are typically used with a tubular member defining a corresponding lumen about 0.01 mm (0.0005 inches) to about 0.04 mm (0.0015 inches) larger than the outer diameter of medical device 100 proximal of change in diameter 831.

Figure 4B illustrates an end view of the apparatus of Figure 4A. The figure includes, from inside to outside (in solid line), electrode 106, electrical insulator 104, the part of distal section 112 proximal of change in diameter 831, tubular member lumen 802, tubular member distal end 801, and tubular member 800. Hidden features shown in broken line include side-port 600 and device lumen 809.

In the embodiment of Figures 4A and 4B, distal tip 403 of the medical device is comprised of electrode 106 which defines a substantially circular cross-section and a circular end-profile. Similar to the embodiments of Figures 3A and 3B, electrode 106 of Figure 4B is at the end of elongate member 102 (or end member 212) and has the same outer diameter as the distal end of the conductive member. Since constant diameter part 836 of reduced diameter distal portion 830 does not substantially taper (the small change in diameter at the distal end of electrical insulator 104 is not taken to be substantial), electrode 106 has a diameter which is substantially equal to the diameter of the part of distal portion 830 which is proximal of electrode 106 (i.e. substantially equal to the diameter of insulated part 834).

Making reference again to Figures 1 to 4, some embodiments of medical device 100 comprise an elongate member 102 having a closed distal end, with the elongate member defining a device lumen 809 and at least one side-port 600 in fluid communication with the device lumen. The elongate member also defines a proximal portion and a distal portion 830, the distal portion extending from the at least one side-port 600 to the distal end of the elongate member. The proximal portion defines a first outer diameter and the distal portion defines a second outer diameter, with the first outer diameter being larger than the second outer diameter, and the second outer diameter being substantially constant. The distal tip of medical device 100 comprises an electrode 106. The diameter of the electrode is substantially equal to the second outer diameter.

Some embodiments of electrode 106 typically create a puncture in tissue with a diameter 10 to 20 percent larger than the electrode. Such a puncture diameter is typically large enough to facilitate passage of the part of medical device proximal of change of diameter 831 (i.e. the larger diameter portion of medical device) through the tissue puncture, and to start advancing a dilator over medical device 100 and through the tissue.

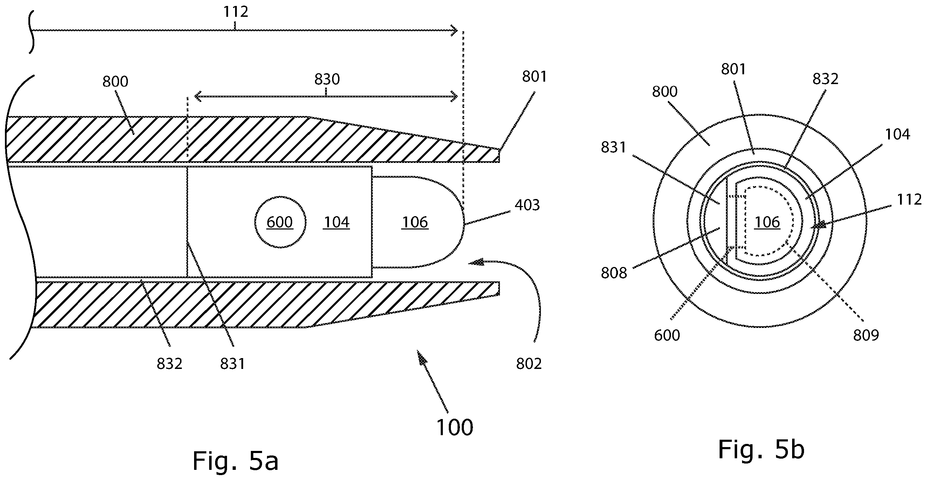

Figures 5A to 5D illustrate a medical device 100 according to the present invention wherein distal portion 830 has a non-circular cross section. In Figures 5A and 5B, distal portion 830 (including electrode 106 and insulated part 834 (Fig. 4a)) defines a substantially flat outer surface portion. The body of medical device 100 defines device lumen 809 (shown in broken line in Figure 5B), and side-port 600 in fluid communication with the lumen. Reduced outer diameter distal portion 830 of the body extends between side-port 600 and distal tip 403 of the medical device whereby the outer surface of medical device 100, in combination with tubular member 800 can provide a conduit 808. While Figure 5A illustrates a portion of reduced outer diameter distal portion 830 extending proximally from side-port 600 to change in diameter 831, some alternative embodiments do not include this portion, i.e., change in diameter 831 is adjacent side-port 600.

The embodiment of conduit 808 in Figure 5B is shown as having an end-view shape of a portion of circle. The reduced outer diameter is substantially constant longitudinally along distal portion 830, with the exceptions of the distal end of electrical insulator 104 and the hemispherical-shaped distal tip of electrode 106. A cross-section of the electrode 106 is substantially identical to a cross-section of the part of the distal portion 830 which is proximal of the electrode.

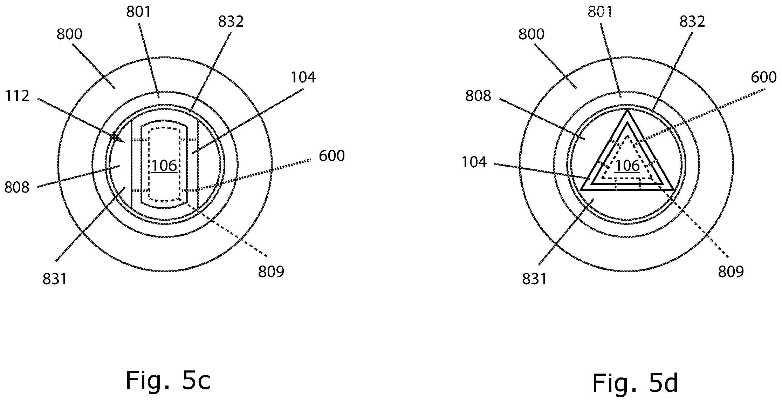

Figure 5C illustrates an alternative embodiment with two flat outer surfaces and two corresponding side-ports. Figure 5D illustrates another alternative embodiment with three flat outer surfaces and three corresponding side-ports. Further alternative embodiments are similar to the embodiments of Figures 5B, 5C and 5D, except instead of the flat outer surfaces, the devices have corresponding outer surfaces that are convexly curved to provide a larger device lumen 809.

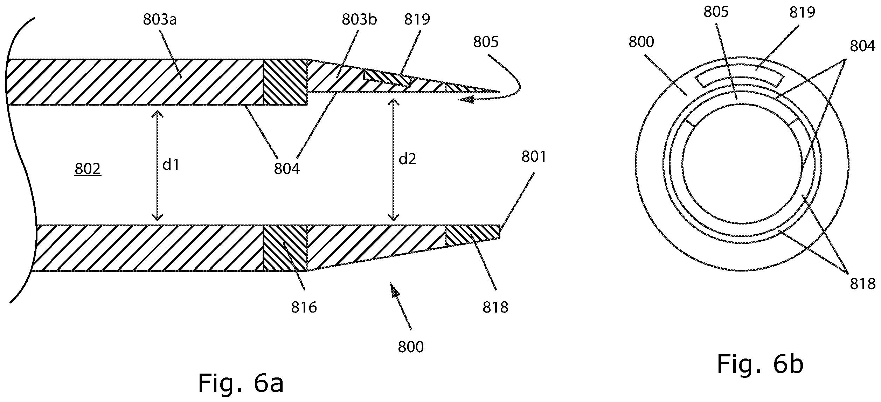

Figure 6A and 6B illustrate an embodiment of a tubular member 800 for use with a medical device 100 having a side-port 600. The body of tubular member 800 defines a lumen such that tubular member proximal region 803a has a first inner diameter d1, and tubular member distal region 803b has at least a portion of it defining a second inner diameter d2, wherein the second inner diameter d2 is greater than the first inner diameter d1, and wherein the tubular member distal region 803b extends to the tubular member distal end 801.

The embodiment of Figure 6B includes the tubular member distal region 803b (i.e. the increased diameter portion with the second inner diameter d2) extending circumferentially over less than 360 degrees of the circumference of the tubular member. Tubular member inner surface 804 defines a tubular member channel 805 which, in the example of Figure 6B, extends circumferentially approximately 90 degrees. In some alternative embodiments, tubular member distal region 803b extends 360 degrees of the circumference of the tubular body.

The embodiment of Figures 6A and 6B includes tubular member proximal marker 816 at the proximal end of the distal region, and tubular member distal marker 818 at the distal end of distal region 803b. Alternative embodiments have only one of the distal region markers or neither distal region marker. The embodiment of Figures 6A and 6B also includes a side marker 819, which is operable to be used as an orientation marker for aligning the tubular member distal region 803b (i.e. the increased diameter portion) with the side-port 600 of a medical device 100 positioned inside the tubular member.

One embodiment is a dilator comprising a tubular member defining a lumen in fluid communication with a distal end aperture, a proximal region having a first inner diameter, and a distal region having an increased diameter portion. The increased diameter portion extends proximally from a distal end of the dilator and defines a substantially longitudinally constant second inner diameter that is greater than the first inner diameter.

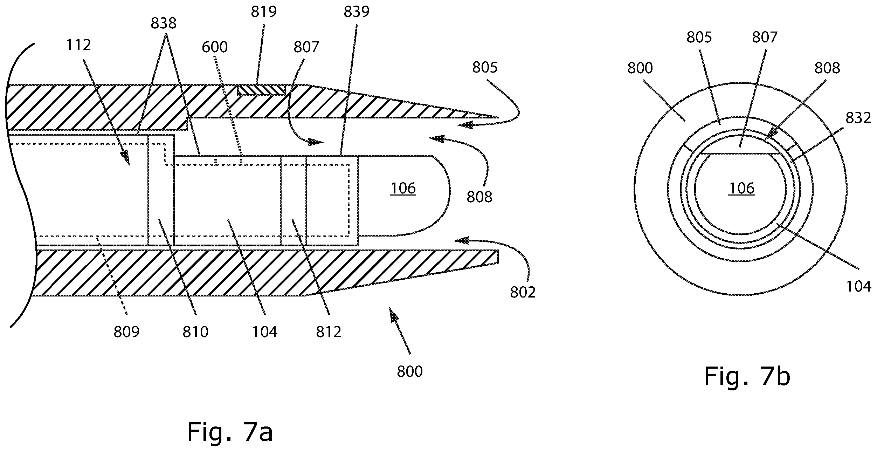

The embodiment of Figures 7A and 7B is a kit comprising a tubular member 800 and a medical device 100, operable to be combined to form an apparatus. Tubular member 800 defines a tubular member lumen 802 for receiving medical device 100. Medical device 100 defines a device lumen 809 in fluid communication with a side-port 600, and comprises a medical device proximal region 838 proximal of the side-port, and a medical device distal region 839 distal of the side-port. Medical device 100 and tubular member 800 are configured for cooperatively forming a conduit 808 between an outer surface of medical device distal region 839 and an inner surface of tubular member 800. In the example of Figure 7A, conduit 808 is formed both proximal and distal of side-port 600, while in alternative embodiments it is only formed distal of the side-port. In typical use, conduit 808 is formed at least between the side-port and a distal end of the tubular member when medical device 100 is inserted and positioned within tubular member lumen 802.

The apparatus of Figure 7A includes both a tubular member channel 805 and a medical device channel 807. Conduit 808 is comprised of both tubular member channel 805 and a medical device channel 807. In typical embodiments, at least some of the length of conduit 808 has a constant cross-sectional configuration, which reduces turbulence and facilitates laminar flow, which in turn facilitates forwards injection of a fluid. Some alternative embodiments include a tubular member channel 805 but not a medical device channel 807, and some other alternative embodiments include a medical device channel 807 but not a tubular member channel 805.

Some embodiments of the medical device and the tubular member further comprise corresponding markers for aligning the side-port of the medical device within the tubular member lumen to form said conduit. In the example of Figure 7, medical device 100 includes medical device proximal marker 810 and medical device distal marker 812, while tubular member 800 includes side marker 819. In some embodiments of the kit, the corresponding markers are configured for longitudinally aligning the side-port within the tubular member lumen. In the example of Figure 7, side-port 600, which is equidistant between medical device proximal marker 810 and medical device distal marker 812, can be longitudinally aligned with side marker 819 by positioning side marker 819 between medical device proximal marker 810 and medical device distal marker 812.

In some embodiments of the kit, the corresponding markers are configured for rotationally aligning the side-port within the tubular member lumen. In the example of Figure 7, side-port 600 can be rotationally aligned with side marker 819 of tubular member 800 by comparing the relatively larger diameter medical device proximal marker 810 with the smaller diameter medical device distal marker 812, which thereby aligns side-port 600 with tubular member channel 805. Alternative embodiments of medical device 100 include a side-marker on the same side as side-port 600, or on the side opposite to the side-port, to facilitate rotational positioning. Further details regarding markers are found in U.S. patent 4,774,949, issued Oct. 4, 1988 to Fogarty.

An embodiment of a kit comprises a tubular member defining a tubular member lumen in fluid communication with a distal end aperture, and a medical device having a closed distal end. The medical device comprises a device lumen in fluid communication with at least one side-port, and a distal portion extending from the at least one side-port to a distal end of the medical device. Medical device and tubular member are configured to cooperatively form a conduit between an outer surface of the distal portion and an inner surface of the tubular member when the medical device is inserted within the tubular member lumen. The conduit extends at least between the side-port and the distal end aperture for enabling fluid communication between the side-port and an environment external to the distal end aperture.

In a specific embodiment of a kit, end member 212 has an on outer diameter proximal of change in diameter 831 of about 0.032 inches (about 0.81 mm), and an outer diameter at reduced diameter distal portion 830 of about 0.020 inches (about 0.51 mm) to about 0.025 inches (about 0.64 mm). End member 212 is used with a tubular member defining a lumen about 0.0325 inches (0.82 mm) to about 0.0335 inches (0.85 mm).

Referring to Figure 8, systems for use with the medical device 100 typically comprise a generator 700 and, in some embodiments, a grounding pad 702, external tubing 706, a pressure transducer 708, and/or a source of fluid 712.

Referring to Figure 8, as mentioned herein above, in order to measure pressure at the distal region 202 (Fig. 10) of the medical device 100, an external pressure transducer may be coupled to the medical device 100. In the example of Figure 8, an adapter 705 is operatively coupled to the external tubing 706, which is operatively coupled to an external pressure transducer 708. The adapter 705 is structured to couple to adapter 704 when in use. In some examples, adapters 704 and 705 comprise male and female Luer locks or other connectors, adapted to readily couple and decouple to/from each other. In use, tubing 706 and 508 may be flushed with saline or another suitable fluid to remove air bubbles prior to measuring pressure. When medical device 100 is positioned in a vessel, conduit, or cavity of a body, fluid adjacent the distal region 202 (Fig. 10) exerts pressure through the side-port(s) 600 on fluid within the lumen 208, which in turn exerts pressure on fluid in tubing 508 and 706, which further exerts pressure on external pressure transducer 708. The side-port(s) 600 and the lumen 208 thus provide a pressure sensor in the form of a pressure transmitting lumen for coupling to a pressure transducer.

The external pressure transducer 708 produces a signal that varies as a function of the pressure it senses. The external pressure transducer 708 is electrically coupled to a pressure monitoring system 710 that is operative to convert the signal provided by the transducer 708 and display, for example, a pressure contour as a function of time. Thus, pressure is optionally measured and/or recorded and, in accordance with one example of a method aspect as described further herein below, used to determine a position of the distal region 202. In those embodiments of the medical device 100 that do not comprise a lumen in fluid communication with the outside environment, a pressure transducer may be mounted at or proximate to the distal section 112 of the medical device 100 and coupled to a pressure monitoring system, for example, via an electrical connection.

As previously mentioned, for some embodiments the medical device 100 is operatively coupled to a source of fluid 712 for delivering various fluids to the medical device 100 and thereby to a surrounding environment. The source of fluid 712 may be, for example, an IV bag or a syringe. The source of fluid 712 may be operatively coupled to the lumen 208 via the tubing 508 and the adapter 704, as mentioned herein above. Alternatively, or in addition, some embodiments include the medical device 100 being operatively coupled to an aspiration device for removing material from the patient's body through one or more of the side-ports 600.

In one broad aspect, the medical apparatus is used in a method of establishing a conduit for fluid communication for a medical device 100, the medical device defining a device lumen 809 in fluid communication with a side-port 600. Making reference to Figures 4 to 9, the method comprises the steps of (a) inserting a medical device 100 having at least one side-port 600 into a tubular member 800, and (b) cooperatively defining a conduit 808 for fluid communication by positioning the side-port 600 of the medical device 100 at a location of the tubular member 800 where a space exists between the side-port 600 and a tubular member inner surface 804, the space extending at least between the side-port 600 and a distal end of the tubular member.

In some embodiments of the broad aspect, the medical device comprises a medical device proximal marker 810 proximal of the side-port, and a medical device distal marker 812 distal of the side-port, and step (b) includes visualizing at least one of the proximal marker and the distal marker to position the medical device. In some such embodiments, step (b) comprises positioning side-port 600 within tubular member lumen 802, for example, by using a medical device proximal marker 810 and a medical device distal marker 812. In such examples of the method, it is not necessary for distal tip 403 to be inside of tubular member lumen 802. In some examples of the method, the medical device further comprises a side-port marker wherein the side-port marker and the side-port are equidistant from a tip of the medical device, and wherein step (b) includes visualizing the side-port marker to position the medical device. In some other embodiments, step (b) comprises positioning distal portion 830 of distal section 112 within tubular member lumen 802, which inherently positions the side-port in the tubular member lumen. In some examples of the method, step (b) includes aligning a distal tip 403 of the medical device with the tubular member distal end 801.

Some embodiments of the broad aspect further comprise a step (c) of delivering fluid through the side-port 600, wherein the fluid is a contrast fluid 814 and wherein step (c) includes delivering the contrast fluid distally through the distal end of the tubular member. Some such embodiments further comprise a step of delivering electrical energy to puncture tissue before the contrast fluid is delivered. Some embodiments comprise a step (d) of delivering electrical energy through the medical device to create a puncture through a tissue after the contrast fluid is delivered.

In some embodiments, the tissue comprises a septum of a heart, and step (c) comprises staining the septum by delivering contrast fluid through the side-port.

In some embodiments of the broad aspect, the side-port 600 and the device lumen 809 together comprise a pressure transmitting lumen, and the method further comprises a step (c) of measuring a pressure of an environment external to the distal end using the side-port and the conduit. Some such embodiments further comprise a step (d) of delivering fluid through the side-port.

Some embodiments of the broad aspect further comprise a step (c) of withdrawing fluid through the side-port 600. In some such embodiments, the fluid is blood.

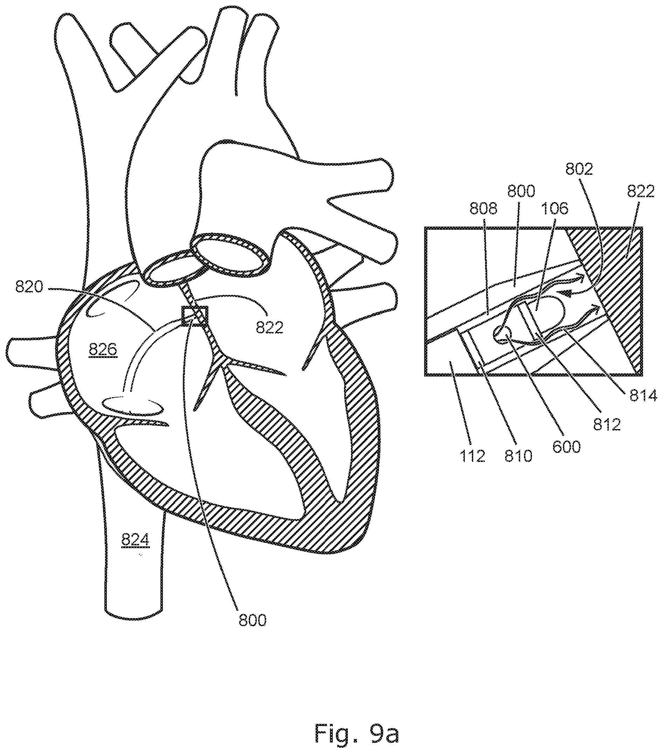

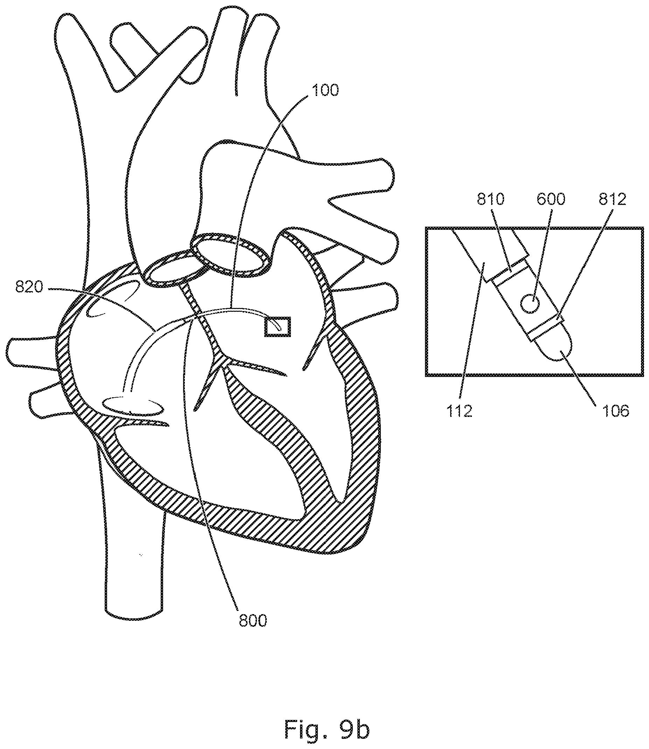

In one example of a method of use, illustrated in Figures 9A and 9B, a target site comprises the atrial septum 822, a tissue within the heart of a patient. In this example, the target site is accessed via the inferior vena cava (IVC), for example, through the femoral vein. The medical device 100 of Figures 9A and 9B is similar to medical device of Figure 4A, except the embodiment of Figure 9 has a medical device proximal marker 810 and a medical device distal marker 812.

The example of the method includes a user advancing sheath 820 and a dilator (i.e. tubular member 800) through inferior vena cava 824, and introducing the sheath and tubular member 800 into the right atrium 826 of the heart. An electrosurgical device, for example medical device 100 described herein above, is then introduced into tubular member lumen 802, and advanced toward the heart. In typical examples of the method, these steps are performed with the aid of fluoroscopic imaging.

After inserting medical device 100 into tubular member 800, the user positions the distal end of tubular member 800 against the atrial septum 822 (Figure 9A). Some embodiments of tubular member 800 include markers (Figure 6A). The medical device is then positioned such that electrode 106 is aligned with or slightly proximal of the distal end of tubular member 800 (Figure 9A insert). Medical device proximal marker 810 and medical device distal marker 812 facilitate positioning medical device 100. Tubular member 800 is typically positioned against the fossa ovalis of the atrial septum 822. Referring to the Figure 9A insert, the inner surface of tubular member 800 and the outer surface of medical device 100 define conduit 808 from side-port 600 to the distal end of tubular member lumen 802, which is sealed by atrial septum 822.

Once medical device 100 and tubular member 800 have been positioned, additional steps can be performed, including taking a pressure measurement and/or delivering material to the target site, for example, a contrast agent, through side-port(s) 600. The Figure 9A insert illustrates contrast fluid 814 flowing from side-port 600, through conduit 808, and ending at atrial septum 822, whereby the tissue is stained by the contrast fluid. In alternative examples, electrode 106 is positioned against atrial septum 822 when contrast fluid 814 is delivered. Such steps facilitate the localization of the electrode 106 at the desired target site.

Starting from the position illustrated by the Figure 9A insert, medical device 100 is advanced until electrode 106 contacts atrial septum 822. (Alternative embodiments wherein electrode 106 is positioned against atrial septum 822 when contrast fluid 814 is delivered do not require this repositioning.) With the medical device 100 and the dilator (i.e. tubular member 800) positioned at the target site, energy is delivered from an energy source, through medical device 100, to the target site. The path of energy delivery is through elongate member 102 (or main member 210 and end member 212), to the electrode 106, and into the tissue at the target site. The example of Figure 9A includes delivering energy to vaporize cells in the vicinity of the electrode, thereby creating a void or puncture through the tissue at the target site, and advancing distal section 112 of the medical device 100 at least partially through the puncture. When the distal section 112 has passed through the target tissue and reached the left atrium (Figure 9B), energy delivery is stopped. The side-ports of medical device 100 are uncovered (Figure 9B insert), whereby contrast may be delivered to confirm the position of distal section 112 in the left atrium of the heart. The diameter of the puncture created by the delivery of energy is typically large enough to facilitate advancing distal section 112 of the medical device 100 therethrough and to start advancing a dilator (i.e. tubular member 820).

Referring now to Figure 10A, the elongate member 102 includes a proximal region 200, a distal region 202, a proximal end 204, and a distal end 206. In some embodiments of the disclosure, the elongate member 102 defines a lumen 208, which typically extends substantially between the proximal region 200 and the distal region 202.

The elongate member 102 is typically sized such that the handle 110 remains outside of the patient when the distal end 206 is within the body, for example, adjacent the target site. That is, the proximal end 204 is at a location outside of the body, while the distal end 206 is located within the heart of the patient. Thus, in some embodiments of the disclosure, the length of the elongate member 102, i.e., the sum of the force transmitting length and the distal section length, is between about 30 cm and about 100 cm, depending, for example, on the specific application and/or target site.

The transverse cross-sectional shape of the elongate member 102 may take any suitable configuration. For example, the transverse cross-sectional shape of the elongate member 102 is substantially circular, ovoid, oblong, or polygonal, among other possibilities. Furthermore, in some embodiments, the cross-sectional shape varies along the length of the elongate member 102. For example, in one embodiment, the cross-sectional shape of the proximal region 200 is substantially circular, while the cross-sectional shape of the distal region 202 is substantially ovoid.

In typical embodiments, the outer diameter of the elongate member 102 is sized such that it fits within vessels of the patient's body. For example, in some embodiments, the outer diameter of the elongate member 102 is between about 0.40 mm and about 1.5 mm (i.e. between about 27 Gauge and about 17 Gauge). In some embodiments, the outer diameter of the elongate member 102 varies along the length of the elongate member 102. For example, in some embodiments, the outer diameter of the elongate member 102 tapers from the proximal end 204 towards the distal end 206. In one specific embodiment, the outer diameter of the proximal region 200 of the elongate member 102 is about 1.5 mm. In this embodiment, at a point about 4 cm from the distal end 206, the outer diameter begins to decrease such that the distal end 206 of the elongate member 102 is about 0.7 mm in outer diameter. In a further embodiment, the outer diameter of the elongate member 102 tapers from about 1.3 mm to about 0.8 mm at a distance of about 1.5 mm from the distal end 206. Figure 10B is an example of a taper in elongate member 102 occurring smoothly, for example, over a length of about 4 cm. Figure 10C is an example of a taper occurring more abruptly, for example, over a length of about 1mm or less. The taper may be applied to the elongate member 102 by a variety of methods. In some embodiments, the elongate member 102 is manufactured with the taper already incorporated therein. In other embodiments, the elongate member 102 is manufactured without a taper, and the taper is created by swaging the elongate member down to the required outside diameter, or by machining the distal region 202 such that the outside diameter tapers while the inside diameter remains constant.

In a further embodiment, the elongate member 102 is manufactured from two pieces of material, each having a different diameter, which are joined together. For example, as shown in Figure 10D, the elongate member 102 includes a main member 210 mechanically coupled to the handle (not shown in Figure 10D), the main member 210 having a length of about 50 cm to about 100 cm and an outer diameter of about 1.15 mm to about 1.35 mm. The main member 210 defines a main member lumen 214, as shown in Figure 2E, extending substantially longitudinally therethrough. The main member is co-axially joined to an end member 212, having a length of about 2.5 cm to about 10 cm and an outer diameter of about 0.40 mm to about 0.80 mm. In some examples, the end member 212 is inserted partially into the main member lumen 214, substantially longitudinally opposed to the handle 110. In some embodiments, the electrode 106 is located about the end member, for example, by being mechanically coupled to the end member 212, while in other embodiments the electrode 106 is integral with the end member 212. If the end member 212 defines an end member lumen 216, as seen in Figures 10D and 2E, the end member lumen 216 is in fluid communication with the main member lumen 214, as shown in Figure 2E. The main member 210 and the end member 212 are joined in any suitable manner, for example welding, soldering, friction fitting, or the use of adhesives, among other possibilities. Also, in some embodiments, the main member lumen 214 and the end member lumen 216 have substantially similar diameters, which reduces turbulence in fluids flowing through the main member lumen 214 and the end member lumen 216.

In embodiments of the disclosure wherein the elongate member 102 defines a lumen 208, the wall thickness of the elongate member 102 may vary depending on the application. For example, if a stiffer device is desirable, the wall thickness is typically greater than if more flexibility is desired. In some embodiments, the wall thickness in the force transmitting region is from about 0.05 mm to about 0.40 mm, and remains constant along the length of the elongate member 102. In other embodiments, wherein the elongate member 102 is tapered, the wall thickness of the elongate member 102 varies along the elongate member 102. For example, in some embodiments, the wall thickness in the proximal region 200 is from about 0.1 mm to about 0.4 mm, tapering to a thickness of from about 0.05 mm to about 0.20 mm in the distal region 202. In some embodiments, the wall tapers from inside to outside, thereby maintaining a consistent outer diameter and having a changing inner diameter. Alternative embodiments include the wall tapering from outside to inside, thereby maintaining a consistent inner diameter and having a changing outer diameter. Further alternative embodiments include the wall of the elongate member 102 tapering from both the inside and the outside, for example, by having both diameters decrease such that the wall thickness remains constant. For example, in some embodiments the lumen 208 has a diameter of from about 0.4 mm to about 0.8 mm at the proximal region 200, and tapers to a diameter of from about 0.3 mm to about 0.5 mm at the distal region 202. In other alternative embodiments, the outer diameter decreases while the inner diameter increases, such that the wall tapers from both the inside and the outside.

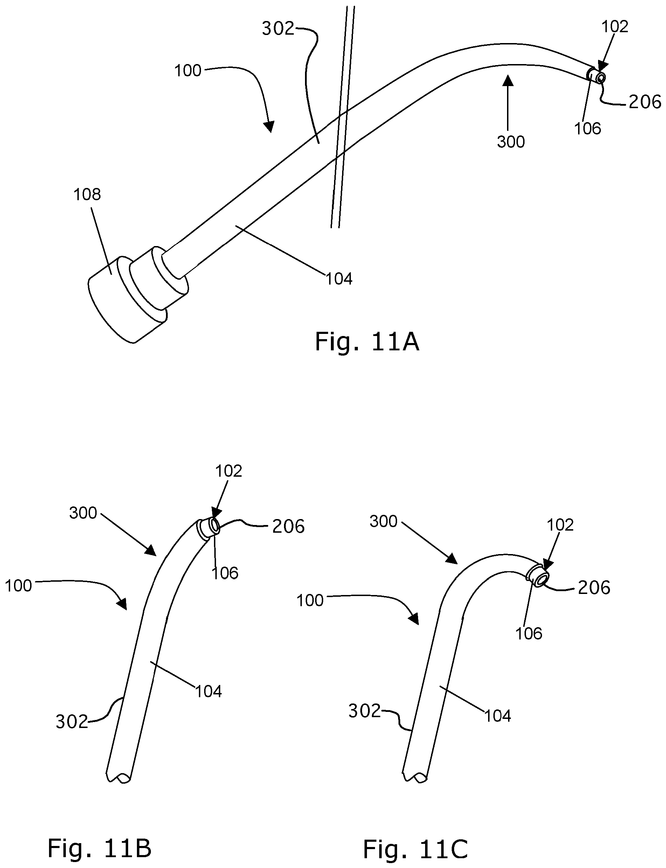

In some embodiments, the elongate member 102, and therefore the medical device 100, are curved or bent, as shown in Figures 11A-11C. As used herein, the terms 'curved' or 'bent' refer to any region of non-linearity, or any deviation from a longitudinal axis of the device, regardless of the angle or length of the curve or bend. The medical device 100 includes a substantially rectilinear section 302 and a curved section 300 extending from the substantially rectilinear section 302. Typically, the curved section 300 is located in the distal region 202 of the elongate member 102, and may occur over various lengths and at various angles. In some examples, curved section 300 has a relatively large radius, for example, between about 10 cm and about 25 cm, and traverses a small portion of a circumference of a circle, for example between about 20 and about 40 degrees, as shown in Figure 11B. In alternative examples, the curved section 300 has a relatively small radius, for example, between about 4 cm and about 7 cm, and traverses a substantially large portion of a circumference of a circle, for example, between about 50 and about 110 degrees, as shown in Figure 11C. In one specific embodiment, the curved section 300 begins about 8.5 cm from the distal end 206 of the elongate member 102, has a radius of about 6 cm, and traverses about 80 degrees of a circumference of a circle. In an alternative embodiment, the curved section has a radius of about 5.4 cm and traverses about 50 degrees of a circumference of a circle. In a further embodiment, the curved section has a radius of about 5.7 cm and traverses about 86 degrees of a circumference of a circle. This configuration helps in positioning the elongate member 102 such that the distal end 206 is substantially perpendicular to the tissue through which the channel is to be created. This perpendicular positioning transmits the most energy when a user exerts a force through the elongate member 102, which provides enhanced feedback to the user.

The curved section 300 may be applied to the elongate member 102 by a variety of methods. For example, in one embodiment, the elongate member 102 is manufactured in a curved mold. In another embodiment, the elongate member 102 is manufactured in a substantially straight shape then placed in a heated mold to force the elongate member 102 to adopt a curved shape. Alternatively, the elongate member 102 is manufactured in a substantially straight shape and is forcibly bent by gripping the elongate member 102 just proximal to the region to be curved and applying force to curve the distal region 202. In an alternative embodiment, the elongate member 102 includes a main member 210 and an end member 212, as described with respect to Figure 10D, which are joined together at an angle (not shown in the drawings). That is, rather than being coaxial, the main member 210 and an end member 212 are joined such that, for example, they are at an angle of 45° with respect to each other.

As mentioned herein above, in some embodiments the proximal region 200 of the elongate member 102 is structured to be coupled to an energy source. To facilitate this coupling, the proximal region 200 may comprise a hub 108 that allows for the energy source to be electrically connected to the elongate member 102. Further details regarding the hub 108 are described herein below. In other embodiments, the proximal region 200 is coupled to an energy source by other methods known to those of skill in the art.

In typical embodiments, the elongate member 102 is made from an electrically conductive material that is biocompatible. As used herein, 'biocompatible' refers to a material that is suitable for use within the body during the course of a surgical procedure. Such materials include stainless steels, copper, titanium and nickel-titanium alloys (for example, NITINOL^{®}), amongst others. Furthermore, in some embodiments, different regions of the elongate member 102 are made from different materials. In an example of the embodiment of Figure 10D, the main member 210 is made from stainless steel such that it provides column strength to a portion of the elongate member 102 (for example, the force transmitting section), and the end member 212 is made out of a nickel-titanium alloy such as NITINOL^{®}, such that it provides flexibility to a portion of the elongate member 102 (for example, the distal section). Embodiments wherein the force transmitting section of the elongate member 102 is manufactured from stainless steel often result in medical device 100 having a similar amount of column strength to a device of the prior art, for example, a mechanical perforator such as a Brockenbrough^{™} needle. This is beneficial in that it provides a familiar 'feel' to users familiar with such devices. In some embodiments comprising a curved or bent elongate member 102, the rectilinear section 302 is made from stainless steel such that it provides column strength to the elongate member 102, and the curved section 300 is made out of a nickel-titanium alloy such as NITINOL^{®}, such that it provides flexibility to the elongate member 102. In addition, the use of NITINOL^{®} for curved section 300 is advantageous as the superelastic properties of this material helps in restoring the shape of the curved section 300 after the curved section 300 is straightened out, for example, when placed within a dilator.

As mentioned herein above, an electrical insulator 104 is disposed on at least a portion of the outer surface of the elongate member 102. In some embodiments, for example as shown in Figure 1, electrical insulator 104 covers the circumference of the elongate member 102 from the proximal region 200 of the elongate member 102 to the distal region 202 of the elongate member 102. In other words, the force transmitting section 114 and distal section 112 are electrically conductive, and the electrical insulator substantially covers the force transmitting section 114 and distal section 112, while the electrode 106 remains substantially uninsulated. When a source of energy is coupled to the proximal region 200 of the elongate member 102, the electrical insulator 104 substantially prevents leakage of energy along the length of the elongate member 102, thus allowing energy to be delivered from the proximal region 200 of the elongate member 102 to the electrode 106.

In embodiments as illustrated in Figure 1, the electrical insulator 104 may extend to different locations on the distal region 202 (Fig. 10), depending on the configuration of the electrode 106. Typically, electrical insulator 104 extends to a proximal end 404 of the electrode 106, which may or may not coincide with the distal end of the elongate member 102. For example, as shown in Figure 3A, the distal-most 1.5 mm of the elongate member 102 serves as at least a portion of the electrode 106. In these embodiments, electrical insulator 104 extends to a point about 1.5 mm proximal to the distal end 206 of the elongate member 102. In the embodiments of Figures 3B - 3C, an external component 400 coupled to the distal end of the elongate member 102 serves as the electrode 106. In such embodiments, the proximal end 404 of the electrode 106 substantially coincides with the distal end 206 of the elongate member 102, and thus the electrical insulator 104 extends to the distal end 206 of the elongate member 102. In some embodiments, the electrical insulator 104 extends beyond the distal end 206 of the elongate member 102, and covers a portion of the external component 400. This typically aids in securing the external component 400 to the elongate member 102. The uncovered portion of the external component 400 can then serve as the electrode 106. In other embodiments, for example as shown in Figure 3A, the distal-most portion of the elongate member 102, as well as an external component 400, serve as the electrode 106. In this embodiment, the electrical insulator 104 extends to a point substantially adjacent to the distal end 206 of the elongate member 102. In one example, the electrical insulator 104 extends to a point about 1.0 mm away from the distal end 206 of the elongate member 102.

The electrical insulator 104 may be one of many biocompatible dielectric materials, including but not limited to, polytetrafluoroethylene (PTFE, Teflon^{®}), parylene, polyimides, polyethylene terepthalate (PET), polyether block amide (PEBAX^{®}), and polyetheretherketone (PEEK^{™}), as well as combinations thereof. The thickness of the electrical insulator 104 may vary depending on the material used. Typically, the thickness of the electrical insulator 104 is from about 0.02 mm to about 0.12 mm.

In some embodiments, the electrical insulator 104 comprises a plurality of dielectric materials. This is useful, for example, in cases where different properties are required for different portions of the electrical insulator 104. In certain applications, for example, substantial heat is generated at the electrode 106. In such applications, a material with a sufficiently high melting point is required for the distal-most portion of the electrical insulator 104, so that this portion of the electrical insulator 104, located adjacent to electrode 106, doesn't melt. Furthermore, in some embodiments, a material with a high dielectric strength is desired for all of, or a portion of, the electrical insulator 104. In some particular embodiments, electrical insulator 104 has a combination of both of the aforementioned features.

With reference now to Figure 2E, the electrical insulator 104 includes a first electrically insulating layer 218 made out of a first electrically insulating material, and a second electrically insulating layer 220 made out of a second electrically insulating material, and being substantially thinner than the first electrically insulating layer 218. The first electrically insulating layer 218 substantially covers the main member 210 substantially adjacent the end member 212, and the second electrically insulating layer 220 substantially covers the end member 212, with the electrode 106 substantially deprived from the second electrically insulating layer 220.. In the illustrated embodiment, the first electrically insulating layer 218 overlaps the second electrically insulating layer 220 about the region of the taper of the elongate member 102. This configuration provides desirable mechanical properties for the medical device 100, as thinner materials are typically less rigid than thicker materials. Also, in some embodiments of the disclosure, the first electrically insulating layer 218 overlaps a portion of the second electrically insulating layer 220. However, in alternative embodiments of the disclosure, the electrical insulator 103 has any other suitable configuration, for example, the first electrically insulating layer 218 and the second electrically insulating layer 220 being made of the same material.

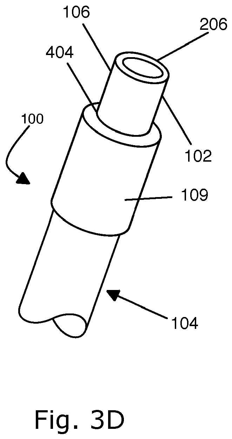

In further embodiments as shown in Figure 3D, a heat shield 109 may be applied to the medical device 100 substantially adjacent to the electrode 106, for example, in order to prevent a distal section of the electrical insulator 104 from melting due to heat generated by the electrode 106,. For example, in some such embodiments, a thermally insulating material, for example Zirconium Oxide or polytetrafluoroethylene (PTFE), is applied over approximately the distal-most 2 cm of the electrical insulator 104. Typically, the heat shield 109 protrudes substantially radially outwardly from the remainder of the distal section 112 and substantially longitudinally from the electrode 106 in a direction leading towards the handle 110.

The electrical insulator 104 may be applied to the elongate member 102 by a variety of methods. For example, if the electrical insulator 104 includes PTFE, it may be provided in the form of heat-shrink tubing, which is placed over the elongate member 102 and subjected to heat to substantially tighten around the elongate member 102. If the electrically insulating material is parylene, for example, it may be applied to the elongate member 102 by vapor deposition. In other embodiments, depending on the specific material used, the electrical insulator 104 may be applied to the elongate member 102 using alternate methods such as dip-coating, co-extrusion, or spraying.

As mentioned herein above, in embodiments of the present invention the elongate member 102 comprises an electrode 106 at the distal region, the electrode 106 configured to create a channel via radiofrequency perforation. As used herein, 'radiofrequency perforation' refers to a procedure in which radiofrequency (RF) electrical energy is applied from a device to a tissue to create a perforation or fenestration through the tissue. Without being limited to a particular theory of operation, it is believed that the RF energy serves to rapidly increase tissue temperature to the extent that water in the intracellular fluid converts to steam, inducing cell lysis as a result of elevated pressure within the cell. Furthermore, electrical breakdown may occur within the cell, wherein the electric field induced by the alternating current exceeds the dielectric strength of the medium located between the radiofrequency perforator and the cell, causing a dielectric breakdown. In addition, mechanical breakdown may occur, wherein alternating current induces stresses on polar molecules in the cell. Upon the occurrence of cell lysis and rupture, a void is created, allowing the device to advance into the tissue with little resistance. In order to increase the current density delivered to the tissue and achieve this effect, the device from which energy is applied, i.e. the electrode, is relatively small, having an electrically exposed surface area of no greater than about 15 mm². In addition, the energy source is capable of applying a high voltage through a high impedance load, as will be discussed further herein below. This is in contrast to RF ablation, whereby a larger-tipped device is utilized to deliver RF energy to a larger region in order to slowly desiccate the tissue. As opposed to RF perforation, which creates a void in the tissue through which the device is advanced, the objective of RF ablation is to create a large, non-penetrating lesion in the tissue, in order to disrupt electrical conduction. Thus, for the purposes of the present invention, the electrode refers to a device which is electrically conductive and exposed, having an exposed surface area of no greater than about 15 mm², and which is operable to delivery energy to create a perforation or fenestration through tissue when coupled to a suitable energy source and positioned at a target site. The perforation is created, for example, by vaporizing intracellular fluid of cells with which it is in contact, such that a void, hole, or channel is created in the tissue located at the target site.

In further embodiments, as shown in Figure 3A, it is desirable for the distal end 206 of the elongate member 102 to be closed. For example, in some embodiments, it is desirable for fluids to be injected radially from the elongate member 102, for example, through side-ports in elongate member 102 substantially without being injected distally from the elongate member 102, as discussed herein below. In these embodiments, a closed distal end 206 facilitates radial injection of fluid while preventing distal injection.

It is a common belief that it is necessary to have a distal opening in order to properly deliver a contrast agent to a target site. However, it was unpredictably found that it is possible to properly operate the medical device 100 in the absence of distal openings. Advantageously, these embodiments reduce the risk that a core of tissue becomes stuck in such a distal opening when creating the channel through the tissue. Avoiding such tissue cores is desirable as they may enter the blood circulation, which creates risks of blocking blood vessels, leading to potentially lethal infarctions.

Thus, as shown in Figure 3A, an external component 400 (Fig. 4b), for example an electrode tip, is operatively coupled to the distal end 206. In this embodiment, the exposed portion of the distal region 202 (Fig. 10), as well as the external component 400, serves as the electrode 106. In such an embodiment, if the outer diameter of the elongate member 102 is 0.7 mm, the external component 400 is a hemisphere having a radius of about 0.35 mm, and the length of the distal-most exposed portion of the elongate member 102 is about 2.0 mm, and then the surface area of the electrode 106 is about 5.2 mm². Alternatively, as shown for example in Figure 2E, the distal end of end member 212 is closed and used as the electrode 106, rather than a separate external component,.

In other embodiments as shown, for example, in Figures 3B and 3C, an electrically conductive and exposed external component 400 is electrically coupled to the distal end of the elongate member 102, such that the external component 400 serves as the electrode 106. In such embodiments, external component 400 is a cylinder having a diameter of between about 0.4 mm and about 1 mm, and a length of about 2 mm. Electrode 106 thus has an exposed surface area of between about 2.6 mm² and about 7.1 mm².

The external component 400 may take a variety of shapes, for example, cylindrical, main, conical, or truncated conical. The distal end of the external component 400 may also have different configuration, for example, rounded, or flat. Furthermore, some embodiments of the external component 400 are made from biocompatible electrically conductive materials, for example, stainless steel. The external component 400 may be coupled to the elongate member 102 by a variety of methods. In one embodiment, external component 400 is welded to the elongate member 102. In another embodiment, external component 400 is soldered to the elongate member 102. In one such embodiment, the solder material itself comprises the external component 400, e.g., an amount of solder is electrically coupled to the elongate member 102 in order to function as at least a portion of the electrode 106. In further embodiments, other methods of coupling external component 400 to the elongate member 102 are used.

In these embodiments, as described herein above, the electrically exposed and conductive surface area of the electrode 106 is no greater than about 15mm². In embodiments wherein the electrical insulator 104 covers a portion of the external component 400, the portion of the external component 400 that is covered by the electrical insulator 104 is not included when determining the surface area of the electrode 106.

Referring again to Figure 3A, in some embodiments, the distal section 112 defines a distal tip 403, the distal tip 403 being substantially atraumatic. In other words, the distal end of the medical device 100 is structured such that it is substantially atraumatic, or blunt. As used herein, the terms 'atraumatic' and 'blunt' refer to a structure that is not sharp, and includes structures that are rounded, obtuse, or flat, amongst others, as shown, for example, in Figure 3A. In embodiments wherein the distal end of the medical device 100 is substantially blunt, the blunt distal end is beneficial for avoiding unwanted damage to non-target areas within the body. That is, if mechanical force is unintentionally applied to the medical device 100 when the distal end of the medical device 100 is located at a non-target tissue, the medical device 100 is less likely to perforate the non-target tissue.

In some embodiments, the distal tip 403 is substantially bullet-shaped, as shown in Figure 2E, which allows the intended user to drag the distal tip 403 across the surface of tissues in the patient's body and to catch on to tissues at the target site. For example, if the target site includes a fossa ovalis, as described further herein below, the bullet-shaped tip may catch on to the fossa ovalis so that longitudinal force applied at a proximal portion of medical device 100 causes the electrode 106 to advance into and through the fossa ovalis rather than slipping out of the fossa ovalis. Because of the tactile feedback provided by the medical device 100, this operation facilitates positioning of the medical device 100 prior to energy delivery to create a channel.

As mentioned herein above, in some embodiments, the medical device 100 comprises a hub 108 coupled to the proximal region. In some embodiments, the hub 108 is part of the handle 110 of the medical device 100, and facilitates the connection of the elongate member 102 to an energy source and a fluid source, for example, a contrast fluid source.

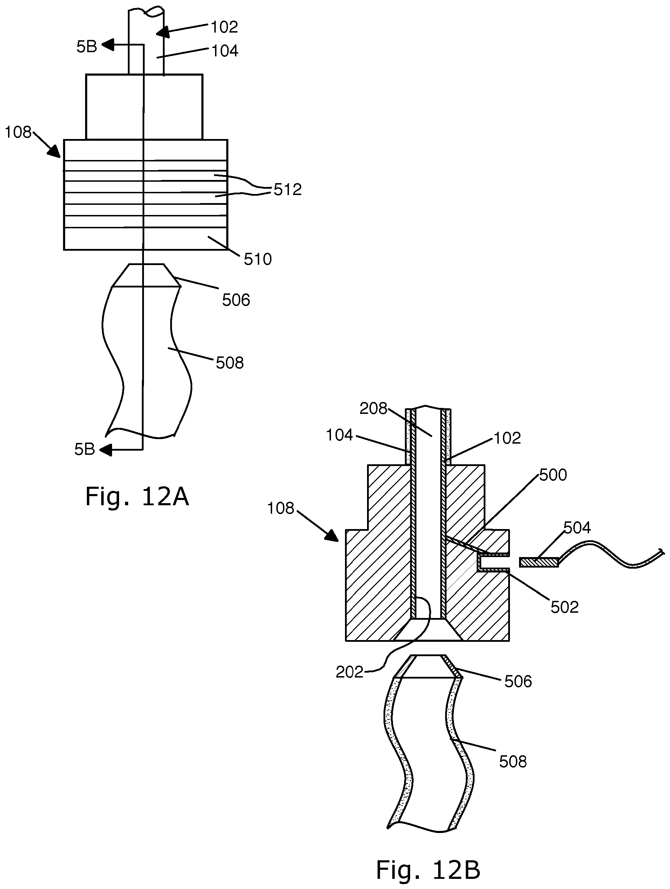

In the embodiment illustrated in Figures 12A and 12B, the proximal region 200 the of the elongate member 102 is electrically coupled to the hub 108, which is structured to electrically couple the elongate member 102 to a source of energy, for example, a radiofrequency generator. In one embodiment, the hub 108 comprises a conductive wire 500 that is connected at one end to the elongate member 102, for example, by welding or brazing. The other end of the wire 500 is coupled to a connector, for example a banana jack 502, that can be electrically coupled to a banana plug 504, which is electrically coupled to a source of energy. Thus, electrical energy may be delivered from the energy source, through plug 504, jack 502, and wire 500 to the elongate member 102 and electrode 106. In other embodiments, other hubs or connectors that allow elongate member 102 to be connected to a source of fluid and a source of energy are used.

In some embodiments, the hub 108 is structured to be operatively coupled to a connector 506, for example a Luer lock, which is connected to tubing 508. Tubing 508 is structured to be operatively coupled at one end to an aspirating device, a source of fluid 712 (for example a syringe), or a pressure sensing device (for example a pressure transducer 708). The other end of tubing 508 may be operatively coupled to the connector 506, such that tubing 508 and lumen 208 are in fluid communication with each other, thus allowing for a flow of fluid between an external device and the lumen 208.

In some embodiments, the hub 108 further comprises one or more curve-direction or orientation indicators 510 that are located on one side of the hub 108 to indicate the direction of the curved section 300. The orientation indicator(s) 510 may comprise inks, etching, or other materials that enhance visualization or tactile sensation.

In some embodiments of the disclosure, the handle 110 includes a relatively large, graspable surface so that tactile feedback can be transmitted relatively efficiently, for example by transmitting vibrations. In some embodiments of the disclosure, the handle 110 includes ridges 512, for example, in the hub 108, which enhance this tactile feedback. The ridges 512 allow the intended user to fully grasp the handle 110 without holding the handle 110 tightly, which facilitates the transmission of this feedback.

In some embodiments of the disclosure, the medical device 100, as shown in Figure 2E, defines a lumen peripheral surface 602 extending substantially peripherally relative to the end member lumen 216, the lumen peripheral surface 602 being substantially covered with a lumen electrically insulating material 604. This configuration prevents or reduces electrical losses from the lumen peripheral surface 602 to any electrically conductive fluid located within the lumen 208. However, in other embodiments of the disclosure, the lumen peripheral surface 602 is not substantially covered with the lumen electrically insulating material 604.

Also, in some embodiments of the disclosure that include the curved section 300, the curved section 300 defines a center of curvature (not shown in the drawings), and the side-port(s) 600 extend from the lumen 208 substantially towards the center of curvature. This configuration substantially prevents the edges of the side-port(s) 600 from catching onto tissues as the tissues are perforated. However, in alternative embodiments of the disclosure, the side-port(s) 600 extend in any other suitable orientation.

In some embodiments, one or more radiopaque markers 714 (as shown in Figure 8) are associated with the medical device 100 to highlight the location of important landmarks on medical device 100. Such landmarks include the location where the elongate member 102 begins to taper, the location of the electrode 106, or the location of any side-port(s) 600. In some embodiments, the entire distal region 202 of the medical device 100 is radiopaque. This can be achieved by filling the electrical insulator 104, for example Pebax^{®}, with a radiopaque filler, for example Bismuth.

In some embodiments, the shape of the medical device 100 may be modifiable. For example, in some applications, it is desired that medical device 100 be capable of changing between a straight configuration, for example as shown in Figure 1, and a curved configuration, for example as shown in Figures 11A - 11C. This may be accomplished by coupling a pull-wire to the medical device 100, such that the distal end of the pull-wire is operatively coupled to the distal region of the medical device 100. When a user applies force to the proximal end of the pull wire, either directly or through an actuating mechanism, the distal region 202 of the medical device 100 is forced to deflect in a particular direction. In other embodiments, other means for modifying the shape of the medical device 100 are used.

In some embodiments, the medical device 100 includes at least one further electrically conductive component, located proximal to the electrode 106. For example, the electrically conductive component may be a metal ring positioned on or around the electrical insulator 104 which has a sufficiently large surface area to be operable as a return electrode. In such an embodiment, the medical device 100 may function in a bipolar manner, whereby electrical energy flows from the electrode 106, through tissue at the target site, to the at least one further electrically conductive component. Furthermore, in such embodiments, the medical device 100 includes at least one electrical conductor, for example a wire, for conducting electrical energy from the at least one further conductive component to a current sink, for example, circuit ground.

In some embodiments, medical device 100 is used in conjunction with a source of radiofrequency energy suitable for perforating material within a patient's body. The source of energy may be a radiofrequency (RF) electrical generator 700, operable in the range of about 100 kHz to about 1000 kHz, and designed to generate a high voltage over a short period of time. More specifically, in some embodiments, the voltage generated by the generator increases from about 0 V (peak-to-peak) to greater than about 75 V (peak-to-peak) in less than about 0.6 seconds. The maximum voltage generated by generator 700 may be between about 180V peak-to-peak and about 3000V peak-to-peak. The waveform generated may vary, and may include, for example, a sine-wave, a rectangular-wave, or a pulsed rectangular wave, amongst others. During delivery of radiofrequency energy, the impedance load may increase due to occurrences such as tissue lesioning near the target-site, or the formation of a vapor layer following cell rupture. In some embodiments, the generator 700 is operable to continue to increase the voltage, even as the impedance load increases. For example, energy may be delivered to a tissue within a body at a voltage that rapidly increases from about 0 V (RMS) to about 220 V (RMS) for a period of between about 0.5 seconds and about 5 seconds.

Without being limited to a particular theory of operation, it is believed that under particular circumstances, as mentioned herein above, dielectric breakdown and arcing occur upon the delivery of radiofrequency energy, whereby polar molecules are pulled apart. The combination of these factors may result in the creation of an insulative vapor layer around the electrode, therein resulting in an increase in impedance, for example, the impedance may increase to greater than 4000Ω. In some embodiments, despite this high impedance, the voltage continues to increase. Further increasing the voltage increases the intensity of fulguration, which may be desirable as it allows for an increased perforation rate. An example of an appropriate generator for this application is the BMC RF Perforation Generator (model number RFP-100, Baylis Medical Company, Montreal, Canada). This generator delivers continuous RF energy at about 460 kHz.

In some embodiments, a dispersive electrode or grounding pad 702 is electrically coupled to the generator 700 for contacting or attaching to a patient's body to provide a return path for the RF energy when the generator 700 is operated in a monopolar mode. Alternatively, in embodiments utilizing a bipolar device, as described hereinabove, a grounding pad is not necessary as a return path for the RF energy is provided by the further conductive component.

In the embodiment illustrated in Figures 12A and 12B, the medical device 100 is operatively coupled to the tubing 508 using connector 506 located at the proximal end of the medical device 100. In some embodiments, the tubing 508 is made of a polymeric material such as polyvinylchloride (PVC), or another flexible polymer. Some embodiments include the tubing 508 being operatively coupled to an adapter 704. The adapter is structured to provide a flexible region for the user to handle when releaseably coupling an external pressure transducer, a fluid source, or other devices to the adapter. In some embodiments, couplings between elongate member 102, connector 506, and tubing 508, and between tubing 508 and adapter 704, are temporary couplings such as Luer locks or other releasable components. In alternative embodiments, the couplings are substantially permanent, for example a bonding agent such as a UV curable adhesive, an epoxy, or another type of bonding agent.

In one broad aspect, the electrosurgical medical device 100 is usable to deliver energy to a target site within a patient's body to perforate or create a void or channel in a material at the target site. Further details regarding delivery of energy to a target site within the body may be found in U.S. Patent Applications 10/347,366 (filed on January 21st, 2003), published as US 2004-0143261 A1, 10/760,749 (filed on January 21st, 2004), published as US 2004-0155005 A1, 10/666,288 (filed on September 19th, 2003), published as US 2004-0143262 A1, and 11/265,304 (filed on November 3rd, 2005), published as US 2006-0142756 A1, and U.S. Patent 7,048,733 (Application 10/666,301, filed on September 19th, 2003) and 6,565,562 (issued on May 20th, 2003).

In one specific embodiment, the target site comprises a tissue within the heart of a patient, for example, the atrial septum of the heart. In such an embodiment, the target site may be accessed via the inferior vena cava (IVC), for example, through the femoral vein.

In one such embodiment, an intended user introduces a guidewire into a femoral vein, typically the right femoral vein, and advances it towards the heart. A guiding sheath, for example, a sheath as described in U.S. Patent Application 10/666,288 (filed on September 19th, 2003), published as US 2004-0143262 A1, is then introduced into the femoral vein over the guidewire, and advanced towards the heart. The distal ends of the guidewire and sheath are then positioned in the superior vena cava. These steps may be performed with the aid of fluoroscopic imaging. When the sheath is in position, a dilator, for example the TorFlex^{™} Transseptal Dilator of Baylis Medical Company Inc. (Montreal, Canada), or the dilator as described in U.S. Patent Application No. 11/727,382 (filed on March 26th, 2007), published as US 2007-0185522 A1, is introduced into the sheath and over the guidewire, and advanced through the sheath into the superior vena cava. The sheath aids in preventing the dilator from damaging or puncturing vessel walls, for example, in embodiments comprising a substantially stiff dilator. Alternatively, the dilator may be fully inserted into the sheath prior to entering the body, and both may be advanced simultaneously towards the heart. When the guidewire, sheath, and dilator have been positioned in the superior vena cava, the guidewire is removed from the body, and the sheath and dilator are retracted slightly such that they enter the right atrium of the heart. An electrosurgical device, for example medical device 100 described herein above, is then introduced into the lumen of the dilator, and advanced toward the heart.

In this embodiment, after inserting the electrosurgical device into the dilator, the user positions the distal end of the dilator against the atrial septum. The electrosurgical device is then positioned such that electrode 106 is aligned with or protruding slightly from the distal end of the dilator. When the electrosurgical device and the dilator have been properly positioned, for example, against the fossa ovalis of the atrial septum, a variety of additional steps may be performed. These steps may include measuring one or more properties of the target site, for example, an electrogram or ECG (electrocardiogram) tracing and/or a pressure measurement, or delivering material to the target site, for example, delivering a contrast agent through side-port(s) 600 and/or open distal end 206. Such steps may facilitate the localization of the electrode 106 at the desired target site. In addition, as mentioned herein above, the tactile feedback provided by the proposed medical device 100 is usable to facilitate positioning of the electrode 106 at the desired target site.

With the electrosurgical device and the dilator positioned at the target site, energy is delivered from the energy source, through medical device 100, to the target site. For example, energy is delivered through the elongate member 102, to the electrode 106, and into the tissue at the target site. In some embodiments, the energy is delivered at a power of at least about 5 W at a voltage of at least about 75 V (peak-to-peak), and, as described herein above, functions to vaporize cells in the vicinity of the electrode, thereby creating a void or perforation through the tissue at the target site. If the heart was approached via the inferior vena cava, as described herein above, the user applies force in the substantially cranial direction to the handle 110 of the electrosurgical device as energy is being delivered. The force is then transmitted from the handle to the distal section 112 of the medical device 100, such that the distal section 112 advances at least partially through the perforation. In these embodiments, when the distal section 112 has passed through the target tissue, that is, when it has reached the left atrium, energy delivery is stopped. In some embodiments, the step of delivering energy occurs over a period of between about 1 s and about 5 s.

At this point in the procedure, the diameter of the perforation is typically substantially similar to the outer diameter of the distal section 112. In some examples, the user may wish to enlarge the perforation, such that other devices such as ablation catheters or other surgical devices are able to pass through the perforation. Typically, to do this, the user applies force to the proximal region of the dilator, for example, in the cranial direction if the heart was approached via the inferior vena cava. The force typically causes the distal end of the dilator to enter the perforation and pass through the atrial septum. The electrosurgical device is operable to aid in guiding the dilator through the perforation, by acting as a substantially stiff rail for the dilator. In such embodiments, a curve, for example curved section 300 of the medical device 100, typically assists in anchoring the electrosurgical device in the left atrium. In typical embodiments, as force is applied, portions of the dilator of larger diameter pass through the perforation, thereby dilating, expanding, or enlarging the perforation. In some embodiments, the user also applies torque to aid in maneuvering the dilator. Alternatively, in embodiments wherein the device is tapered, the device may be advanced further into the left atrium, such that larger portions of the device enter and dilate the perforation.

In some embodiments, when the perforation has been dilated to a suitable size, the user stops advancing the dilator. A guiding sheath is then advanced over the dilator through the perforation. In alternative embodiments, the sheath is advanced simultaneously with the dilator. At this point in the procedure, the user may retract the dilator and the electrosurgical device proximally through the sheath, leaving only the sheath in place in the heart. The user is then able to perform a surgical procedure on the left side of the heart via the sheath, for example, introducing a surgical device into the femoral vein through the sheath for performing a surgical procedure to treat electrical or morphological abnormalities within the left side of the heart.

If an apparatus of the present invention, as described herein above, is used to carry out a procedure as described herein, then the user is able to maintain the 'feel' of a mechanical perforator, for example a Brockenbrough^{™} needle, without requiring a sharp tip and large amounts of mechanical force to perforate the atrial septum. Rather, a radiofrequency perforator, for example, the electrode 106, is used to create a void or channel through the atrial septum, as described herein above, while reducing the risk of accidental puncture of non-target tissues.

In other examples, methods of the present disclosure may be used for treatment procedures involving other regions within the body, and the invention is not limited in this regard. For example, rather than the atrial septum, embodiments of devices, systems, and methods of the present disclosure can be used to treat pulmonary atresia. In some such embodiments, a sheath is introduced into the vascular system of a patient and guided to the heart, as described herein above. A dilator is then introduced into the sheath, and advanced towards the heart, where it is positioned against the pulmonary valve. An electrosurgical device comprising an electrode is then introduced into the proximal region of the dilator, and advanced such that it is also positioned against the pulmonary valve. Energy is then delivered from the energy source, through the electrode of the electrosurgical device, to the pulmonary valve, such that a puncture or void is created as described herein above. When the electrosurgical device has passed through the valve, the user is able to apply a force to the proximal region of the dilator, for example, in a substantially cranial direction. The force can be transmitted to the distal region of the dilator such that the distal region of the dilator enters the puncture and advances through the pulmonary valve. As regions of the dilator of larger diameter pass through the puncture, the puncture or channel becomes dilated.

In other applications, embodiments of a device of the present invention can be used to create voids or channels within or through other tissues of the body, for example within or through the myocardium of the heart. In other embodiments, the device is used to create a channel through a fully or partially occluded lumen within the body. Examples of such lumens include, but are not limited to, blood vessels, the bile duct, airways of the respiratory tract, and vessels and/or tubes of the digestive system, the urinary tract and/or the reproductive system. In such embodiments, the device is typically positioned such that an electrode of the device is substantially adjacent the material to be perforated. Energy is then delivered from an energy source, through the electrode 106, to the target site such that a void, puncture, or channel is created in or through the tissue.

This disclosure describes embodiments of a kit and its constituent components which together form an apparatus in which fluid communication between a medical device's lumen and the surrounding environment is provided by a conduit cooperatively defined by the medical device and a tubular member into which the device is inserted. The medical device and tubular member are configured to fit together such that an outer surface of the distal region of the medical device cooperates with an inner surface of the tubular member to define the conduit between the side-port of the medical device and a distal end of the tubular member. The conduit is operable for a variety of applications including injecting fluid, withdrawing fluid, and measuring pressure. Methods of assembling and using the apparatus are described as well.

The embodiments of the disclosure described above are intended to be exemplary only. The scope of the invention is intended to be limited solely by the scope of the appended claims.

Although the invention has been described in conjunction with specific embodiments thereof, it is evident that many alternatives, modifications and variations are apparent to those skilled in the art. Accordingly, it is intended to embrace all such alternatives, modifications, and variations that fall within the scope of the appended claims.

## Claims

1. A kit comprising:
- a tubular member (800) defining a tubular member lumen (805) and a distal end aperture in fluid communication therewith;
- a medical device (100) for enabling fluid flow within the tubular member (800), the medical device (100) comprising:
an elongate member having a closed distal end, the elongate member defining a device lumen and a single side-port (600) in fluid communication with the device lumen, the elongate member defining a proximal portion and a distal portion, the distal portion extending from proximal of the single side-port (600) to the closed distal end of the elongate member;
the proximal portion defining a circular cross section and a first outer diameter, the distal portion at the single side-port defining a non-circular cross section and a second outer diameter, the first outer diameter being larger than the second outer diameter;
a distal tip comprising an electrode (106), wherein the second outer diameter is substantially constant between the single side-port and the electrode;
the elongate member comprising an electrically conductive material and at least one layer of insulation covering the electrically conductive material, the electrically conductive material being electrically exposed to define the electrode (106);
the distal portion (830) defining a substantially flat outer surface portion with the single side-port being located therein and the at least one layer of insulation extending proximally from the electrode such that the single side-port (600) extends through the electrically conductive material and the at least one layer of insulation; and
the elongate member defining a discrete change in diameter between the proximal portion and the distal portion and the single side-port being proximate the discrete change in diameter, whereby the proximal portion is configured to support the tubular member when the medical device is inserted therethrough to prevent the tubular member from obstructing the single side-port;
wherein the medical device and tubular member cooperatively form a conduit (808) between the substantially flat outer surface portion of the distal portion and an inner surface of the tubular member (800) when the medical device (100) is inserted within the tubular member lumen (805), the conduit (808) having a cross-section in the shape of a circular segment and being formed at least between the single side-port and the distal end aperture for enabling fluid communication with minimized or reduced obstruction between the single side-port and an environment external to the distal end aperture, and at least a part of a length of the conduit (808) has a constant cross-sectional configuration.

2. The kit of claim 1, wherein a diameter of the electrode (106) is substantially equal to the second outer diameter.

3. The kit of claim 1, wherein the medical device (100) and the tubular member (800) further comprise corresponding markers for aligning the medical device within the tubular member lumen to form said conduit.

4. The kit of claim 1, wherein the electrically conductive material comprises a metal.

5. The kit of claim 1, further comprising a connector operable to connect the elongate member to a fluid source.

## Patentansprüche

1. Kit, umfassend:
- ein röhrenförmiges Element (800), das ein Lumen (805) des röhrenförmigen Elements und eine distale Endöffnung in Fluidverbindung mit diesem definiert;
- eine medizinische Einrichtung (100) zum Ermöglichen eines Fluidflusses innerhalb des röhrenförmigen Elements (800), wobei die medizinische Einrichtung (100) umfasst:
ein längliches Element mit einem geschlossenen distalen Ende, wobei das längliche Element ein Einrichtungslumen und einen einzelnen Seitenanschluss (600) in Fluidverbindung mit dem Einrichtungslumen definiert, wobei das längliche Element einen proximalen Abschnitt und einen distalen Abschnitt definiert, wobei sich der distale Abschnitt proximal des einzelnen Seitenanschlusses (600) zu dem geschlossenen distalen Ende des länglichen Elements erstreckt;
wobei der proximale Abschnitt einen kreisförmigen Querschnitt und einen ersten Außendurchmesser definiert, wobei der distale Abschnitt an dem einzelnen Seitenanschluss einen nicht kreisförmigen Querschnitt und einen zweiten Außendurchmesser definiert, wobei der erste Außendurchmesser größer ist als der zweite Außendurchmesser;
eine distale Spitze, umfassend eine Elektrode (106), wobei der zweite Außendurchmesser zwischen dem einzelnen Seitenanschluss und der Elektrode im Wesentlichen konstant ist;
wobei das längliche Element ein elektrisch leitendes Material und mindestens eine Isolierschicht umfasst, die das elektrisch leitende Material bedeckt, wobei das elektrisch leitende Material elektrisch freigelegt ist, um die Elektrode (106) zu definieren;
wobei der distale Abschnitt (830) einen im Wesentlichen flachen äußeren Oberflächenabschnitt definiert, wobei sich der einzelne Seitenanschluss darin befindet und die mindestens eine Isolationsschicht sich proximal von der Elektrode erstreckt, so dass sich der einzelne Seitenanschluss (600) durch das elektrisch leitende Material und die mindestens eine Isolationsschicht erstreckt; und
wobei das längliche Element eine diskrete Änderung des Durchmessers zwischen dem proximalen Abschnitt und dem distalen Abschnitt und den einzelnen Seitenanschluss, der in der Nähe der diskreten Änderung des Durchmessers liegt, definiert, wodurch der proximale Abschnitt eingerichtet ist, das röhrenförmige Element zu stützen, wenn die medizinische Einrichtung durch ihn hindurch eingeführt wird, um zu verhindern, dass das röhrenförmige Element den einzelnen Seitenanschluss blockiert;
wobei die medizinische Einrichtung und das röhrenförmige Element zusammen eine Leitung (808) zwischen dem im Wesentlichen flachen äußeren Oberflächenabschnitt des distalen Abschnitts und einer inneren Oberfläche des röhrenförmigen Elements (800) bilden, wenn die medizinische Einrichtung (100) in das Lumen (805) des röhrenförmigen Elements eingeführt ist, wobei die Leitung (808) einen Querschnitt in der Form eines kreisförmigen Segments aufweist und zumindest zwischen dem einzelnen Seitenanschluss und der distalen Endöffnung ausgebildet ist, um eine Fluidverbindung mit minimierter oder reduzierter Blockierung zwischen dem einzelnen Seitenanschluss und einer Umgebung außerhalb der distalen Endöffnung zu ermöglichen, und zumindest ein Teil einer Länge der Leitung (808) eine konstante Querschnittskonfiguration aufweist.

2. Kit nach Anspruch 1, wobei ein Durchmesser der Elektrode (106) im Wesentlichen gleich dem zweiten Außendurchmesser ist.

3. Kit nach Anspruch 1, wobei die medizinische Einrichtung (100) und das röhrenförmige Element (800) ferner entsprechende Markierungen zum Ausrichten der medizinischen Einrichtung innerhalb des Lumens des röhrenförmigen Elements umfassen, um die Leitung zu bilden.

4. Kit nach Anspruch 1, wobei das elektrisch leitende Material ein Metall umfasst.

5. Kit nach Anspruch 1, ferner umfassend ein Verbindungsstück, das zur Verbindung des länglichen Elements mit einer Flüssigkeitsquelle bedient werden kann.

## Revendications

1. Kit comprenant :
- un élément tubulaire (800) qui définit une lumière d'élément tubulaire (805) et une ouverture d'extrémité distale en communication en termes de fluide avec ; et
- un dispositif médical (100) pour permettre un écoulement de fluide à l'intérieur de l'élément tubulaire (800), le dispositif médical (100) comprenant :
un élément allongé qui comporte une extrémité distale fermée, l'élément allongé définissant une lumière de dispositif et un unique orifice latéral (600) en communication en termes de fluide avec la lumière de dispositif, l'élément allongé définissant une partie proximale et une partie distale, la partie distale étant étendue depuis à proximité de l'unique orifice latéral (600) jusqu'à l'extrémité distale fermée de l'élément allongé ;
la partie proximale définissant une section en coupe transversale circulaire et un premier diamètre externe, la partie distale au niveau de l'unique orifice latéral définissant une section en coupe transversale non circulaire et un second diamètre externe, le premier diamètre externe étant plus grand que le second diamètre externe ;
une pointe distale qui comprend une électrode (106), dans lequel le second diamètre externe est sensiblement constant entre l'unique orifice latéral et l'électrode ;
l'élément allongé comprenant un matériau électriquement conducteur et au moins une couche d'isolation qui recouvre le matériau électriquement conducteur, le matériau électriquement conducteur étant exposé électriquement de manière à définir l'électrode (106) ;
la partie distale (830) définissant une partie de surface externe sensiblement plane dans laquelle l'unique orifice latéral est localisé et l'au moins une couche d'isolation étant étendue de façon proximale par rapport à l'électrode de telle sorte que l'unique orifice latéral (600) soit étendu au travers du matériau électriquement conducteur et de l'au moins une couche d'isolation ; et
l'élément allongé définissant une variation discrète en termes de diamètre entre la partie proximale et la partie distale et l'unique orifice latéral étant à proximité de la variation discrète en termes de diamètre, d'où il résulte que la partie proximale est configurée pour supporter l'élément tubulaire lorsque le dispositif médical est inséré au travers afin d'empêcher que l'élément tubulaire n'obstrue l'unique orifice latéral ;
dans lequel le dispositif médical et l'élément tubulaire forment en coopération un conduit (808) entre la partie de surface externe sensiblement plane de la partie distale et une surface interne de l'élément tubulaire (800) lorsque le dispositif médical (100) est inséré à l'intérieur de la lumière d'élément tubulaire (805), le conduit (808) présentant une section en coupe transversale selon la forme d'un segment circulaire et étant formé au moins entre l'unique orifice latéral et l'ouverture d'extrémité distale pour permettre une communication en termes de fluide selon une obstruction minimisée ou réduite entre l'unique orifice latéral et un environnement qui est externe par rapport à l'ouverture d'extrémité distale, et au moins une partie d'une longueur du conduit (808) présente une configuration en coupe transversale constante.

2. Kit selon la revendication 1, dans lequel un diamètre de l'électrode (106) est sensiblement égal au second diamètre externe.

3. Kit selon la revendication 1, dans lequel le dispositif médical (100) et l'élément tubulaire (800) comprennent en outre des marqueurs en correspondance pour aligner le dispositif médical à l'intérieur de la lumière d'élément tubulaire afin de former ledit conduit.

4. Kit selon la revendication 1, dans lequel le matériau électriquement conducteur comprend un métal.

5. Kit selon la revendication 1, comprenant en outre un connecteur qui peut être rendu opérationnel pour connecter l'élément allongé à une source de fluide.
